# EUROPEAN PATENT APPLICATION

(11) **EP 1 953 228 A1**
(43) Date of publication of application: **06.08.2008**
(21) Application number: 07101745.3
(22) Date of filing: 05.02.2007
(51) Int. Cl.: C12N 15/11, C12N 5/06

(54) **Method to diminish cd83 expression in monocyte-derived dendritic cells, t-cells and b-cells by RNAi**

(71) Applicant: Friedrich-Alexander-Universität Erlangen-Nürnberg, 91054 Erlangen (DE)
(72) Inventor: Turza, Nadine, 91083 Baiersdorf (DE); Steinkasserer, Alexander, Prof. Dr., 91207 Erlangen (DE); Prechtel, Alexander, Dr., 91207 Lauf (DE)
(74) Representative: von Kreisler Selting Werner

(57) **Abstract**

The present invention provides a RNAi method for the specific reduction of CD83 surface expression by dendritic cells (DCs), T-cells and B-cells, especially by DCs. This method can be used to inhibit the T cell activating ability of DCs and for reduction or suppression of T-cell proliferation in immune response. The invention further provides a RNAi agent targeted to the CD83 transcript and dendritic cells obtainable by the method. A method and a medicament for the treatment of a clinical condition which is based on the negative effect of a CD83 mediated interaction between mature DCs and T-cells in a mammalian subject, and a method for decreasing the immunogenicity and rejection potential of a graft using said RNAi agent are also provided.

## Description

The present invention provides a RNAi method for the specific reduction of CD83 surface expression by dendritic cells (DCs), T-cells and B-cells, especially by DCs. This method can be used to inhibit the T cell activating ability of DCs and for reduction or suppression of T-cell proliferation in immune response. The invention further provides a RNAi agent targeted to the CD83 transcript and dendritic cells obtainable by the method. A method and a medicament for the treatment of a clinical condition which is based on the negative effect of a CD83 mediated interaction between mature DCs and T-cells in a mammalian subject, and a method for decreasing the immunogenicity and rejection potential of a graft using said RNAi agent are also provided.

### Background of the Invention

Antigen Presenting Cells (APCs) are characterized by specialized features such as pathogen recognition, antigen processing, migratory capacity and surface expression of co-stimulatory molecules. Immune responses are initiated in the T-cell areas of secondary lymphoid organs, where APCs such as mature dendritic cells (mDCs) present processed antigens to naïve T-lymphocytes (1). Mature human Dendritic Cells are the most powerful antigen-presenting cells known today and have the unique ability to induce primary immune responses. For the induction of proper immune responses, a direct contact between the dendritic cell (DC) and the T-cell is necessary. Therefore, naïve T-cells form an immunological synapse with the APC, where T-cell-receptors (TCR), MHC complexes, and co-stimulatory molecules get in close contact, surrounded by a ring of adhesion molecules (2). The signals triggered inside the T-cell by these interactions are influenced by three factors: i) the duration of the cell-cell contact; ii) the number of MHC complexes that induce a signal, and iii) the number of co-stimulatory signals that enhance the message (3). The provision of co-stimulatory signals in the form of cytokines (such as IL-2 or IL-12) and membrane-bound ligands (such as CD80 (B7-1) and CD86 (B7-2)) by mDCs regulate T-cell activation and differentiation (4,5).

Amongst many other surface molecules that are up-regulated during maturation of DCs, one membrane-bound glycoprotein and member of the IgG superfamily is of extraordinary importance: CD83. This molecule is today's best known cell surface marker for fully mature human DCs that are able to induce immune responses (1). It is strongly upregulated during maturation, together with co-stimulatory molecules such as CD80 and CD86. Although a pre-form of CD83 has been found inside monocytes, macrophages and immature DCs (iDCs), it is only stably expressed on mDCs (6) and some activated T-cells and B-cells (7). Two different isoforms of this CD83 have been reported *in vivo* so far: a membrane-bound form (mCD83; (8-10)) and a soluble form (sCD83; (11,12)). The latter one is thought to be generated by proteolytic cleavage of the mCD83 (12). However, a precise mechanism for the generation of sCD83 has not been described yet.

Both forms of CD83 seem to have completely different functions: while sCD83 seems to act as an immunosuppressive molecule, whose main function is to interfere with DC mediated immune responses (12,21-23), the membrane bound CD83 plays an important role during the induction of cellular immune responses. To simulate the membrane-bound form of CD83, Scholler and co-workers fused the extracellular domain of CD83 to an Ig-domain (i.e. CD83-Ig) and co-immobilized it with anti-CD3 antibodies (the latter are needed for activation of T cells via the CD3 complex; Bockenstedt, L.K. et al., Rheum. Dis. Clin. North Am. 13(3):411-430 (1987); (17)). Interestingly, only this co-immobilized protein induced strong proliferation of PBMCs, while CD83-Ig alone failed to do so (17). Furthermore, the ratio of CD8⁺ to CD4⁺ T-cells increased by factor 2.5, indicating a specific function of mCD83 in the induction of T-lymphocyte differentiation and the enhancement of immune responses (17,24). Identification of CD137 as a cofactor for increased antitumor immunity of CD83 expressing cells essentially confirmed this observation (25). However, CD83 seems not only to be important during T-cell activation, but is also of particular importance during T-cell development in the thymus. Fujimoto and co-workers demonstrated by using CD83 deficient (CD83^{-/-}) knockout mice that without CD83 significantly less CD4 single-positive T-cells developed (26). The specific modulation of CD83 surface expression would therefore be very useful in exploiting its role during induction of immune responses and tolerance as well as during prevention of autoimmune diseases and control of allergy.

Maturation is the critical step for DCs to become potent APCs and to be able to activate naïve T-cells. During this maturation process, specific surface molecules such as CD83 or MHC class II are strongly upregulated together with co-stimulatory molecules such as CD80 and CD86. The inhibition of entire DC maturation leads to tolerance induction and suppression of unwanted immune responses (1,36).

Of outmost importance is the fact that several viruses use CD83 as a target to prevent the host's immune response. The infection of iDCs with Herpes Simplex Virus Type 1 (HSV-1) prevented up-regulation of CD83 on the cell surface during maturation (13), while the infection of mDCs led to the down-modulation and intracellular degradation of mCD83 (14). In both cases, the loss of the membrane-bound isoform correlated with a significantly reduced capacity to stimulate T-cells. For another member of the *Herpesviridae* family, the Human Cytomegalovirus (HCMV), Senechal and co-workers reported that the infection leads to a conversion of the mCD83 into the soluble form of CD83 (15). The infection of mDCs with the HCMV led to the shedding of CD83 from surface, although the intracellular expression of the protein remained constant. The resulting soluble CD83 accumulated in the supernatant of the cells and was also able to interfere with T-cell activating properties of DCs (15). In this respect it is noteworthy that sCD83 has been described to impair the activation of T-cells by mDCs (15-17).

Furthermore, it has been shown that DC function can be modulated by interfering with the processing of the CD83 mRNA. A first hint came from the observation that expression of CD83 goes hand in hand with the expression of eukaryotic initiation factor 5A (eIF-5A; (18)), a protein involved in the export of cellular mRNAs from the nucleus to the cytoplasm via the specific CRM-1 nucleo-cytoplasmic mRNA export pathway (19). The expression level of eIF-5A is very low in iDC but increases during DC maturation. For its biological function as a mRNA transporter, eIF-5A has to undergo posttranscriptional processing, namely hypusine modification. So far, eIF-5A is the only known cellular protein that contains the unusual amino acid hypusine (20). When this posttranscriptional modification is blocked by a low molecular weight inhibitor (GC7), the specific CRM-1 nucleo-cytoplasmic mRNA export is blocked and iDCs completely fail to up-regulate CD83 during maturation, whereas other molecules like CD80 or CD86 are not influenced at all. The loss of CD83 surface expression is due to a block of CD83 mRNA export from the nucleus into the cytoplasm and leads in consequence to a reduction of the ability of DCs to induce allogenic T-cell proliferation, i.e. is further correlated with the loss of T-cell stimulatory capacity (18).

A similar observation on DCs *in vitro* and *in vivo* has been described by Zinser and colleagues. The addition of CNI-1493 (also an inhibitor of the CRM1 export pathway (37)) reduced the expression of CD83 during maturation and DC-mediated T-cell stimulation (38). It additionally reduced the clinical symptoms of an experimental autoimmune encephalomyelitis (EAE) model when applied in an early therapeutic setting (38).

Thus, when CD83 surface expression was inhibited by interference with the messenger RNA export or by infection with certain viruses, DCs showed a dramatically reduced capability to induce T-cell proliferation. However, in these cases side effects on other cellular functions may occur. When blocking the intracellular mRNA transport by GC7 or CNI-1493, it cannot be ruled out that these drugs may also affect the metabolism of additional mRNAs, leading to impairment of the DCs or unwanted effects on T-cell proliferation. Viruses (especially HSV-1) have been shown to act on several stages to evade the immune system. HSV-1 for example is able to block maturation of DCs (13) and prevents mature DCs from migration to the areas of T-cell activation, i.e. into the secondary lymphoid organs (39).

A common and well established way to specifically control gene activity is the method of RNA interference (RNAi) (Fire, A. et al., Nature 391:806-811 (1998); Zamore, P.D. et al., Cell 101:25-33 (2000); Elbashir, S.M. et al., Nature 411:494-498 (2001)). RNAi is a conserved pathway present in most eukaryotes wherein dsRNA triggers a series of biochemical events that culminates in seqeunce-specific suppression of gene expression. In mammals, long dsRNAs are cleaved by the RNase III class endoribonuclease Dicer ("diced") into 21 to 23 base duplexes having 2-base 3'-overhangs. These species, called siRNAs, enter the RNA induced silencing complex (RISC) and serve as a sequence-specific guide to target degradation of complementary mRNA.

RNAi by small interfering RNA (siRNA) or small hairpin RNA (shRNA) is a mechanism for posttranscriptional gene silencing in which the steady-state level of a specific mRNA is specifically decreased (27,28). For instance, a process leading to the specific degradation of mRNAs in a target cell involves the formation of the RNA-induced silencing complex (RISC) under participation of a siRNA. The RISC then targets the messenger RNA (mRNA) for degradation (Radhakrishnan, P.I. et al., Drugs of the Future 28(1): 51-59 (2003); Sontheimer, E.J., Nat. Rev. Mol. Cell Biol. 6(2): 127-138 (2005)). Typically, siRNAs for use in RNAi are chemically synthesized as 21mers with a central 19 bp duplex and symmetric 2-base 3'-overhangs on the termini. These duplexes are transfected into cell lines, directly mimicking the products made by Dicer *in vivo.* Thus, siRNA duplexes of 21 nucleotides - which can be easily produced in large scale - represent an efficient way to induce sequence specific gene silencing (29,30). Modification of DC function by siRNA technology has been proven to be an important and efficient way to investigate DC's biology (for examples see (31-33)).

The potential of siRNAs for use as therapeutic agents to reduce activity of specific gene products is also receiving considerable attention. Successful knockdown of gene expression *in vivo* has already been demonstrated by several groups.

WO 2005/085443 describes the use of RNAi agents (e.g. siRNA) for the treatment of diseases mediated by IgE, namely allergic rhinitis and asthma.

WO 03/104456 describes the alteration of gene expression in antigen presenting cells such as DCs and their use in methods and compositions to alter T cell activity for the treatment of a variety of immune disorders. Said alteration may be achieved by RNAi via siRNA. However, successful targeting in DCs is only demonstrated for IL-12 and TNF-alpha.

The problem to be solved by present invention is the provision of an efficient and selective method to reduce or knockdown CD83 surface expression in DCs, T-cells and B-cells, but especially in DCs. A further aim of present invention is the provision of a specific method for the treatment of diseases caused by the dysfunction of a cellular immune response, especially of autoimmune diseases, allergic reactions and for the prophylaxis of graft rejection.

### Summary of the Invention

Present invention provides an efficient method to specifically influence CD83 surface expression in DCs, T-cells and B-cells by the use of RNA interference (RNAi). The method is especially efficient in DCs. A long lasting silencing effect for CD83 is achieved by using said method. This was for the first time realized without the use of agents that might cause unwanted side effects, such as low molecular weight inhibitors or viruses, and is therefore a suitable method to influence DCs' biology. The CD83 surface expression is reduced specifically without interfering with the expression of other molecules.

Finally, it is confirmed that CD83 functions as an enhancer during the stimulation of T-cells and significantly increases DC-mediated T-cell proliferation. Mature dendritic Cells with diminished mCD83 which were obtained by the RNAi method of present invention show reduced capacity to stimulate allogeneic T-cells. Thus, inhibition of CD83 surface expression by RNAi can be used for the treatment of diseases caused by the dysfunction of a cellular immune response, especially of autoimmune diseases, allergic reactions and for the prophylaxis of graft rejection.

Thus, the present invention provides
(1) a method for the specific reduction of CD83 surface expression by dendritic cells (DCs), comprising the transfection of a starting DC population with a RNAi agent (preferably an siRNA) which is (i) targeted to a transcript encoding the membrane associated CD83 (mCD83) and (ii) capable of selectively reducing or inhibiting the mCD83 expression on the DC surface;
(2) The method of (1) above, wherein the starting DC population consists of immature DC (iDC);
(3) the method of (2) above, which further comprises a maturation step after the transfection of the iDC to obtain mature DC;
(4) the method of any one of (1) to (3) above which is suitable for reduction or inhibition of the T cell activating ability of the DCs, and for reduction or suppression of T-cell proliferation in immune response;
(5) a RNAi agent which is as defined in (1) above or is a nucleic acid that comprises a template for transcription of one or more RNA molecules that hybridize or self-hybridize to form a siRNA or shRNA targeted to the CD83 transcript;
(6) dendritic cells obtainable by the method as defined in any one of (1) to (4) above;
(7) a method for the treatment or prevention of a disease or clinical condition which is caused by the dysfunction or undesired function of a cellular immune response mediated by DCs, especially by a CD83 mediated interaction between mature DCs and T-cells in a mammalian subject, said method comprising administering a therapeutically effective amount of a RNAi agent as defined in (5) above or a dendritic cell as defined in (6) above to said subject;
(8) a medicament for the treatment of a disease or clinical condition as defined in (7) above, which comprises the RNAi agent of (5) above or the dendritic cells of (6) above; and
(9) a method for decreasing the immunogenicity and rejection potential of a graft, said method comprising treating the prospective recipient and/or the graft itself with a composition that reduces or suppresses CD83 expression on the surface of DC, said composition comprising an RNAi agent as defined in (5) above.
   In the method of embodiment (1), preferably a small-interfering RNA (siRNA) is targeted against CD83. Electroporation is preferred for the delivery of the duplex into the cells. Furthermore, freshly prepared, immature DCs are the best target for the application of a CD83 knockdown RNAi agent.
   The clinical condition in embodiments (7) and (8) is preferably an autoimmune disease, an allergic reaction or a graft rejection.
   The mDC83 surface expression can furthermore be suppressed in T-cells and/or B-cells using the RNAi method of present invention. Thus, present invention further provides
(10) a method for the specific reduction of CD83 surface expression by T-cells or B-cells, comprising the transfection of a starting T-cell or B-cell population with a RNAi agent which is (i) targeted to a transcript encoding the membrane associated CD83 (mCD83) and (ii) capable of selectively reducing or inhibiting the mCD83 expression on the cell surface;
(11) T-cells or B-cells obtainable by the method as defined in (11) above;
(12) a method for the treatment or prevention of a disease or clinical condition which is caused by the dysfunction or undesired function of a cellular immune response mediated by T-cells and/or B-cells, said method comprising administering a therapeutically effective amount of a RNAi agent as defined in (5) above or cells as defined in (11) above to said subject; and
(13) a medicament for the treatment of a disease or clinical condition as defined in (12) above, which comprises cells as defined in (11) above.

### Description of the Figures

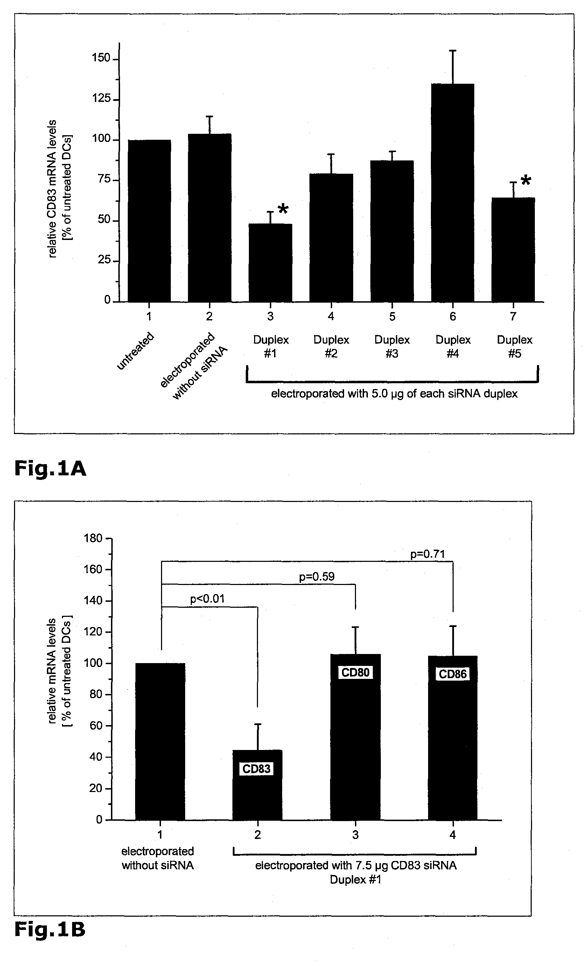

Fig. 1: Identification of an efficient and specific siRNA duplex targeted against CD83.
(A) iDCs were electroporated with 5.0 µg of different CD83-siRNA duplexes and maturation was induced 4 h after electroporation. After 24 h maturation time, total RNA was isolated, reverse transcribed and Real-Time-PCRs were performed for CD83 mRNA as the target and β-glucuronidase as an internal control. The normalized ratio of CD83 to β-glucuronidase in untreated cells was set to 100% (column 1). iDCs electroporated without any siRNA demonstrated no significant downregulation of the CD83 messenger (column 2). A significant effect (p<0.01; marked by asterisks) on CD83 mRNA could be detected in case of duplex #1 and #5 (columns 3 and 7). The specific mRNA level was reduced up to 50%. The data represent the mean ± SD of three independent experiments. Changes were considered as significant if p<0.01.
(B) Additional Real-Time-PCRs were performed to quantify the mRNA amounts of the typical DC surface markers CD80 and CD86. The normalized ratios of iDCs electroporated without any siRNA were set to 100% and compared to cells that were electroporated with 7.5 µg of duplex #1. Only in the case of CD83 mRNA a significant downregulation was observed (column 1). Neither CD80 (column 2) nor CD86 (column 3) were influenced by the siRNA duplex, demonstrating the specificity of the knockdown. The data represent the mean ± SD of three independent experiments. Changes were considered as significant if p<0.01.

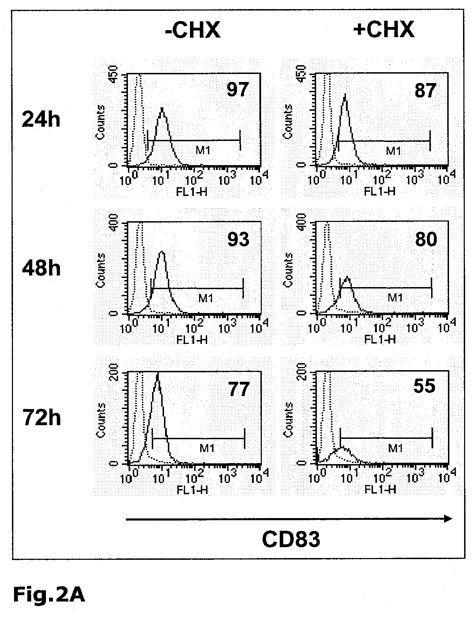

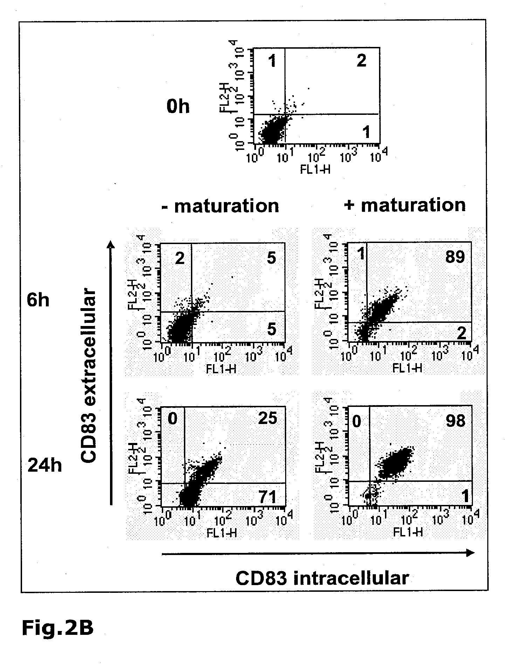

Fig. 2: CD83 is stable over a long time and its expression is rapidly induced upon maturation.
(A) Mature DCs were either incubated for 24 h, 48 h or 72 h in the absence (left column) or presence (right column) of 10 µg/ml cycloheximide (CHX). Surface expression of CD83 was determined by FACS analysis at the indicated time points. After 24 h still 87% of the CHX treated cells were positive for CD83, while 97% of the untreated cells showed CD83 surface expression (upper panels). After 48 h 80% of the CHX-treated DCs express CD83 on their surface, while 93% of the untreated population were CD83 positive (middle panels). 72 h after CHX-incubation, 55% of the cells are positive for CD83, compared to 77% positive cells without the inhibitor (lower panels). After 72 h most of the DCs showed severe damage due to the CHX treatment. The data are representative for four independent experiments with cells from different donors.
(B) Immature DCs were simultaneously analysed for intra- and extracellular CD83 expression in the absence or presence of maturation stimuli. Freshly prepared iDCs (topmost panel) do not express large amounts of CD83, as only 2% of the population was double-positive for intra- and extracellular CD83. Induction of maturation (right column) led to rapid expression of both intra- and extracellular CD83: 89% of the cells were double-positive after 6 h and 98% after 24 h. Although no maturation stimuli were present in the media, iDCs accumulated intracellular CD83, probably due to the mechanical treatment/stress. After 24 h, 96% of the cells express intracellular CD83 (left column, lower panel, upper and lower right quadrant; representing fluorescence channel 1). The data are representative for three independent experiments with cells from different donors.

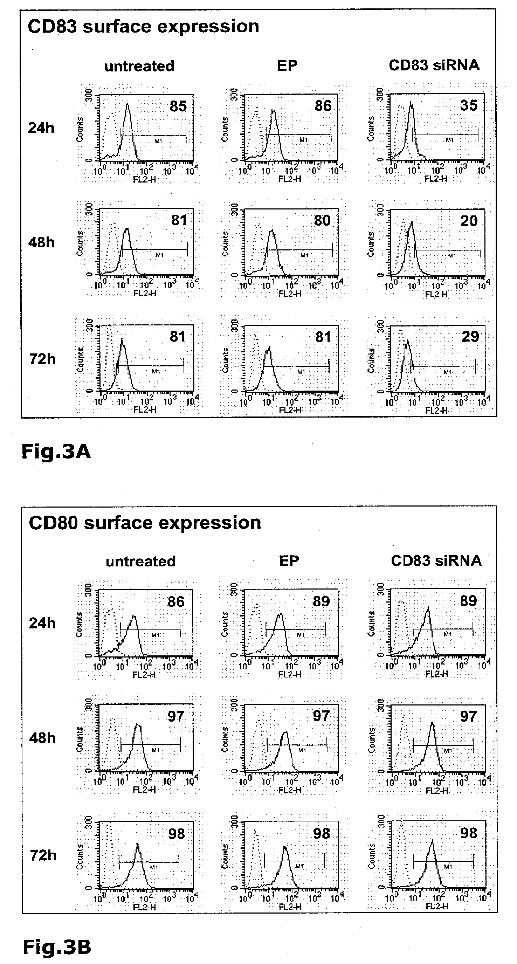

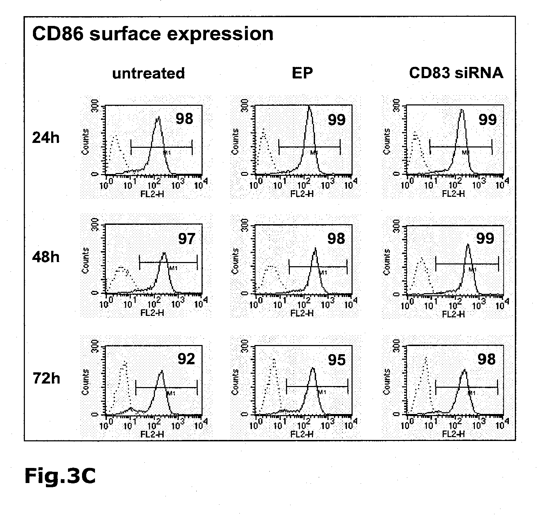

Fig. 3: CD83 knockdown is functional, specific and long lasting on protein level; FACS analyses of the surface expression of CD83 (A), CD80 (B) and CD86 (C). Immature DCs were either left untreated (A-C; left column), mock-electroporated (A-C; middle column) or electroporated with 7.5 µg of CD83 siRNA duplex #1 (A-C; right column). While the surface expression of CD80 and CD86 were not influenced at any condition, CD83 was efficiently knocked down from cell's surface (3A; right column; untreated cells: 81-85%; mock-electroporated cells: 80-86%; CD83 duplex #1 electroporated: 20-35%). The silencing of CD83 was also a long lasting effect, because after 72 h still only 29% of the cells expressed CD83 on their surface (3A; right column, lower panel). The data are representative for five independent experiments with cells from different donors. The levels of CD83 surface expression might vary from donor to donor, however, the knockdown effect was always similar when compared with untreated cells.

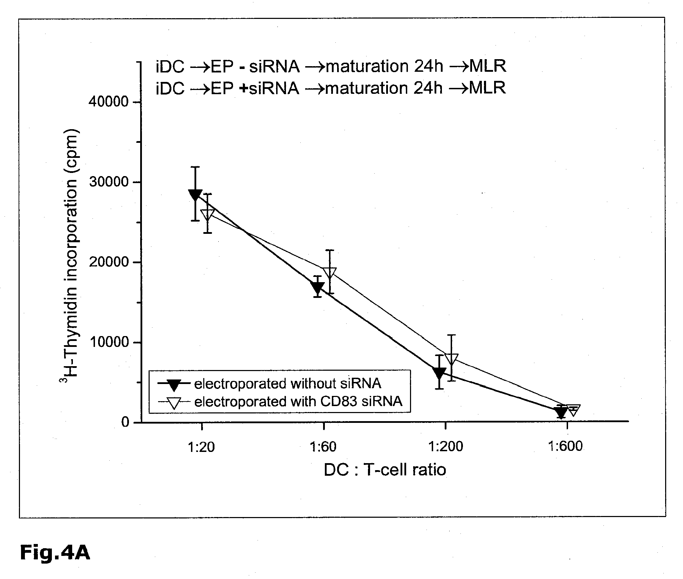

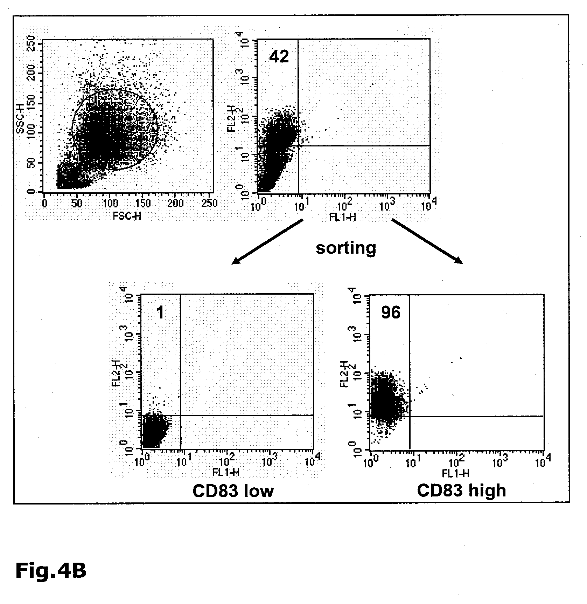

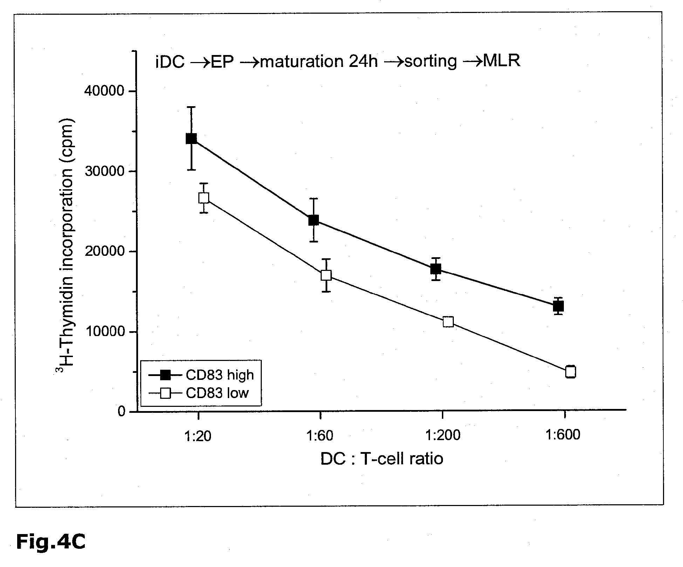

Fig. 4: CD83low mature DCs show reduced capacity to stimulate allogenic T-cells.
(A) Immature DCs were electroporated with 7.5 µg of CD83 siRNA duplex #1, incubated for 4 h, matured for 24 h and finally seeded together with T-cells for 72 h. Cells were then pulsed with [³H]-thymidine for 24 h, harvested and the filters were counted. The data demonstrated that cells that were electroporated with CD83 siRNA (open triangles) showed similar capacity to stimulate T-cells when compared with cells that were mock-electroporated and treated the same way afterwards (filled triangles). These results are representative for three independent experiments with cells from different donors. The data represent the mean ± SD of three setups of the same experiment.
(B) MoFlo^{®} sorting of DCs: Immature DCs that were electroporated with 7.5 µg duplex #1, incubated for 4 h and finally matured for 24 h were analyzed by FACS (upper panel). 42% of the siRNA treated population was still positive for CD83. Afterwards the cells were sorted into a CD83low population (cells that showed the same fluorescence as the respective isotype-control) and a CD83high population by MoFlo^{®}. The resulting populations were again analysed by FACS: in the CD83low population (lower left panel) only 1% of the cells were CD83-positive, whereas 96% of the CD83high population were CD83-positive (lower right panel).
(C) Mixed leukocyte reaction (MLR) with CD83high and CD83low DCs revealed a reduced DC mediated T-cell proliferation in the CD83 specific siRNA treated DC population. MLR were performed as described in Fig. 4A. mDCs that had high surface expression of CD83 (filled quadrat) showed a significant higher capability to induce T-cell proliferation than the CD83low population (open quadrat). Mature state of the CD83low population was verified by FACS for typical DC surface markers (CD80, CD86, MHC II; data not shown). These results are representative for three independent experiments with cells from different donors. The data represent the mean ± SD of three setups of the same experiment.

### Sequence Listing, Free Text

| SEQ ID NO: | description |
|---|---|
| 1 | CD83 mRNA, version NM_004233.3 |
| 2 | CD83 mRNA, version NM_004233.1 |
| 3 | CD83 mRNA, version NM_004233.2 |
| 4 | sense strand siRNA #1 |
| 5 | antisense strand siRNA #1 |
| 6 | sense strand siRNA #2 |
| 7 | antisense strand siRNA #2 |
| 8 | sense strand siRNA #3 |
| 9 | antisense strand siRNA #3 |
| 10 | sense strand siRNA #4 |
| 11 | antisense strand siRNA #4 |
| 12 | sense strand siRNA #5 |
| 13 | antisense strand siRNA #5 |
| 14 | sense (forward) primer CD80 |
| 15 | antisense (reverse) primer CD80 |
| 16 | sense primer CD83 |
| 17 | antisense primer CD83 |
| 18 | sense primer CD86 |
| 19 | antisense primer CD86 |
| 20 | sense primer β-glucuronidase |
| 21 | antisense primer β-glucuronidase |

### Abbreviations and Definitions

The following abbreviations are used: DC, monocyte-derived Dendritic Cell; iDC, immature DC; mDC, mature DC; FACS, Fluorescence Activated Cell Sorting; RT-PCR, Real-Time Polymerase Chain Reaction; mRNA, messenger ribonucleic acid; sCD83, soluble CD83; mCD83, membrane-bound CD83; MLR, allogenic Mixed Leukocyte Reaction; PBMC, peripheral blood mononuclear cell; RNAi, RNA interference.

Some of the terms used in present application are defined as follows:

A "nucleotide" according to the present invention is a nucleotide occurring in natural DNA or RNA not having undergone chemical modification.

An "oligonucleotide" according to the present invention is a double or single stranded nucleic acid molecule consisting of from 10 to 100, preferably from 19 to 30, more preferably from 19 to 27, yet more preferably from 21 to 27, and most preferably 21 nucleotides per single strand. Said oligonucleotide preferably consists of ribonucleotides or a combination of ribonucleotides and deoxyribonucleotides.

The terms "CD83 transcript" and "CD83 mRNA" are used as synonyms in present invention and, if not indicated otherwise, mean the pre-mRNA transcript of the CD83 gene and the corresponding mature mRNA which comprise, preferably consist of the 5' UTR, CDS and 3' UTR of the transcription product resulting from the transcription of the CD83 gene. In a preferred aspect of present invention, they mean the mRNA encoding mCD83.

"RNAi" (RNA interference) designates the interference with a target mRNA which leads to reduction or inhibition of gene expression either by blockage of mRNA expression/translation (RNAi agent: miRNA = microRNA) or by posttransciptional mRNA degradation (RNAi agent: siRNA) in a target cell (Agrawal, N. et al., Microbiol. Mol. Biol. Rev. 2003; 67(4):657-85). In more detail, RNAi is a mechanism in molecular biology where the presence of certain fragments of double stranded RNA (dsRNA) interferes with the expression of a particular gene which shares a homologous sequence with the dsRNA. It appears that the RNAi machinery, once it finds a dsRNA, cuts it up with the Dicer enzyme, at which point the dsRNA interacts with the RNA-induced silencing complex (RISC). Then, the RNA is unwound to a ssRNA form, and the RISC is considered activated. The ssRNA will then hybridize with mRNA. If the base-pairing is perfect or near-perfect, the argonaute protein component of the RISC will cleave the mRNA. If the base-pairing is imperfect, RISC is thought to downregulate genes primarily through translation inhibition.

An "RNAi agent" according to the present invention is to be understood as a compound which has the ability to initiate RNAi for a specific mRNA to which it is targeted (target mRNA). It is usually a siRNA or shRNA.

The term "siRNA" according to the present invention refers to an oligonucleotide duplex formed by two single oligonucleotides usually designated as "sense strand and "antisense strand". The siRNA may comprise single stranded parts at its 5' and/or 3' ends ("overhangs") and preferably does comprise 3'-overhangs, more preferably 2-base 3'-overhangs. It has RNAi activity. Each strand of the siRNA comprises from 19 to 30, preferably from 19 to 27, more preferably from 21 to 27, yet more preferably 21 nucleotides (compare Rose, S.D. et al., Nucl. Acid Res. 33:4140-4156 (2005)).

The term "shRNA" ("short hairpin RNA") according to the present invention refers to a partly doublestranded oligonucleotide comprising a single stranded loop connecting the doublestranded regions. shRNA has RNAi activity. It comprises from 40 to 70, preferably from 50 to 60, and most preferably 55 nucleotides.

A "precursor" of a siRNA or shRNA is an oligonucleotide according to the present invention, in particular a completely or partly dsRNA, which is cut enzymatically, e.g. by the enzyme complex Dicer, to generate said siRNA or shRNA.

The siRNAs, shRNAs and their precursors according to the present invention may be obtained commercially, from a suitable cell after introduction of an appropriately engineered vector or plasmid, or by artificial synthesis using methods and protocols well-known in the art (see e.g. Radhakrishnan, P.I. et al., Drugs of the Future 28(1): 51-59 (2003) and references cited therein).

As used herein, the term "substantially homologous" refers to oligonucleotide molecules that generally demonstrate a substantial percent sequence identity with the sequences of the siRNAs provided herein when they are aligned. Of particular interest are oligonucleotide molecules which possess RNAi activity targeted to the CD83 transcript and have at least about 85% sequence identity, at least about 90% sequence identity, or even greater sequence identity, such as 98% or 99% sequence identity with the sequences of the siRNAs or their respective target sequences described herein.

A "target sequence" is a segment of the target mRNA of the RNAi agent. One strand of the duplex portion of the RNAi agent (the "antisense strand") is complementary to said target sequence or at least substantially homologous to such complementary sequence. Under physiologic conditions, once unwound, the single stranded antisense strand guides RISC to mRNA that has a complementary sequence, which results in the endonucleolytic cleavage of the target mRNA. The "sense strand" of the RNAi agent seems to be relevant for uptake of the siRNA into the RISC (Jackson, A.L. et al., RNA 12:1197-1205 (2006)). The function of sense and antisense strand is discussed in Dykxhoorn, D.M. and Lieberman, J., Annu. Rev. Med. 56:401-423 (2005); Dykxhoorn, D.M. and Lieberman, J., Cell 126:231-235 (2006); and Dykxhoorn, D.M. et al., Nature Rev. Mol. Cell Biol. 4:457-467 (2003). Thus, the functionality of an siRNA is based mainly on the interaction of its antisense strand with the target mRNA and the RISC complex.

The terms "reduction", "reduce" and "reduced" refer to a decrease of the respective parameter (e.g., the CD83 surface expression, the capacity to stimulate T-cells) in comparison to the same parameter as determined for reference systems (usually systems using DC cells which were not subject to RNAi). They pertain to any amount of decrease, but preferably to a decrease of at least 40 % and up to 100 % compared with the reference cells, more preferably to a decrease of at least 70 %. Reduction levels of 100 % are most preferred. A 100 % reduction of CD 83 expression is synonymous with a complete inhibition or suppression of CD83 in the measured DCs. The term "knockdown" which is sometimes used in present text encompasses reduction and inhibition of gene expression.

In the context of present invention, the reduction of CD83 expression on the mRNA level and on the protein level is of specific interest. In the first aspect, the reduction of the target mRNA level in comparison to a reference level (which is represented by the mRNA level in above reference system or by the initial mRNA level before the RNAi) is relevant. This reduction is known in the art as "transcript knockdown". A transcript knockdown of at least 70 % is generally acknowledged as satisfactory for RNAi in the art. The transcript knockdown in the context of present invention is preferably at least 70 %, more preferably at least 85 %. It may even reach or exceed 90 %.

In the second aspect, the reduction of the CD83 protein level, especially of the mCD83 protein level, in comparison to the reference level (which is represented by the protein level in above reference system) is of specific interest. This reduction may be designated as "protein knockdown". A protein knockdown of at least 50 % is generally acknowledged as satisfactory for RNAi in the art. The protein knockdown in the context of present invention is preferably at least 70 %, more preferably at least 85 %. It may even reach or exceed 90 %.

The term "transfection" usually means the transfer, for the purposes of genomic integration, of foreign DNA into a host cell. The traditional microbiological usage of this term implied that the DNA being transferred was derived from a virus. The definition as stated here is that which is in use in the up to date state of the art to describe the general transfer of DNA irrespective of its source and of the transfer means. The term is used synonymously with "transformation" throughout present description. The same applies to the verbs "transfect" and "transform".

### Detailed Description of the Invention

Present invention provides for the first time an efficient method to knockdown, i.e. reduce or even inhibit, CD83 surface expression in DCs, T-cells and B-cells, especially in DCs, by the use of RNAi. The RNAi may be effected by any RNAi conferring compound including siRNA and shRNA, but the use of siRNA is preferred.

The RNAi agent of present invention may be used for downregulating the expression of CD83 in mammalian (especially human) cells (especially DCs, T cells and B cells, most preferably DCs) on the RNA and/or protein level. A downregulation on the protein level is preferred.

In more detail, in the method of embodiment (1) the RNAi agent preferably comprises a nucleic acid duplex formed by a sense and an antisense strand, more preferably a ribonucleic acid duplex. The duplex portion is preferably from 17 to 28 nucleotides (nt), more preferably from 17 to 25 nt, most preferably 19 nt long. A RNAi agent longer than 21 nt is suitable for present invention, because it is diced *in vivo* into shorter products (siRNA) (see, e.g., Rose, S.D. et al., Nucl. Acid Res. 33:4140-4156 (2005)).

The RNAi agent in the method of embodiment (1) is preferably an oligonucleotide, more preferably is an siRNA or shRNA sequence. Even more preferred is an isolated siRNA or shRNA sequence, which is not provided within a plasmid or vector or by a siRNA or shRNA precursor.

In an especially preferred aspect of present invention, the RNAi agent is a small interfering RNA (siRNA), a short hairpin RNA (shRNA) or a precursor thereof, preferably is a siRNA.

Thus, the most preferred RNAi agent suitable for the method of embodiment (1) is a siRNA which comprises from 19 to 30, yet more preferably from 19 to 27, still more preferably from 21 to 27, and most preferably 21 nucleotides per single strand and whose duplex portion has the length as outlined above.

There are some criteria for RNAi agents which - when met - generally enhance stability and specifity of said RNAi agents. Design considerations for maximally active siRNAs that have a low potential for off-target, unintended gene targeting are described in Dykxhoorn, D. M. and Lieberman, J., Annu. Rev. Biomed. Eng. 8:377-402 (2006). Furthermore, RNAi agent design which leads to enhanced stability and nuclease resistance is described in Pei, Y and Tuschl, T, Nature Methods 3:670-676 (2006). RNAi agents meeting one or more of the criteria given in this literature are preferred in the context of present invention.

The RNAi agent may furthermore in one preferred aspect be modified by position specific chemical modification as described in Jackson, A. L. et al., RNA, 12:1197-1205 (2006) or any other modification which reduces "off-target" transcript silencing.

In the method of embodiment (1), the target sequence in the CD83 transcript may be any segment of the CD83 transcript (the CD83 mRNA including the CDR and the 3' and 5' untranslated regions (UTR)) or a sequence substantially homologous to such segment. In one preferred aspect, the target sequence has a length from 17 to 28 nt, more preferably from 17 to 25 nt, most preferably of 19 nt. The target sequence is located in the 3'UTR, the Coding Sequence (CDS) or the 5'UTR. Preferably, it is located in the segment of the CD83 transcript consisting of the 3'UTR and the CDS of the CD83 transcript, more precisely in the region from nt 179 to nt 2478 of the CD83 mRNA as represented by SEQ ID NO:1 (CDS: nt 179 to 796; 3'UTR: nt 797 to 2478; 5'UTR: nt 1 to 796). More preferably, it is located in the segment consisting of the 3'end of the CDS and the 3'UTR of the CD83 transcript represented by nt 550 to 2478 of SEQ ID NO:1, most preferably in the following regions: nt 590 to nt 1450 or nt 2000 to nt 2478 of SEQ ID NO:1. Most preferably, it is located in the region represented by nt 590 to 793 or nt 1250 to 1400 of SEQ ID NO: 1.

The region represented by nt 590 to 793 of SEQ ID NO:1 is of relevance because it comprises the SL1 and SL2 region of the CD83 mRNA (Prechtel, A.T. et al., J. Biol. Chem. 2006, 281:10912-25; US-A-2002165186). SL1 is represented by nt 590 to 647 of SEQ ID NO:1, SL2 is represented by nt 648 to 793 of SEQ ID NO:1. SL2 binds to HuR, a RNA-binding protein which upon binding of CD83 mRNA enhances CD83 expression (Prechtel, A.T. et al., J. Biol. Chem. 2006, 281:10912-25). Notably, quite a few of the siRNA molecules designed for the CD83 RNA by known algorithms (see below) are situated inside this region. Inside this region, the SL2 region is even more preferred due to its binding to HuR.

The target sequence in the CD83 transcript and in consequence the sequence of the RNAi agent which is complementary to said target sequence may be selected using known methods for RNAi agent design, especially siRNA design. A suitable guide for siRNA selection, representative siRNA sequence selection web tools and criteria for the selection of effective and specific siRNA are given in Pei, Y and Tuschl, T, Nature Methods 3:670-676 (2006). Further indications on designing effective siRNA are given in Dykxhoorn, D. M. et al., Nature Reviews 4:457-467 (2003). SiRNA fulfilling some or all of the criteria given in these two papers are preferred in the context of present invention.

Most of said RNAi design methods are based on a sequence walk on the target transcript in 1-nucleotide-steps. E.g. one may start with a sequence part of 19 nt length (as initial target sequence) at any given site of the transcript, move the 19 frame 1 nt into the 5 '- or 3 '-direction (second target sequence) and repeat this several times. Of course, the desired length of the target sequence may be varied. Thus, all theoretically accessible siRNAs for a target sequence may be determined. However, siRNA molecules whose target sequence is comprised within one of the preferred regions of the CD83 transcript as outlined above are of course preferred in the context of present invention.

Preferably, the method for RNAi design is the use of the Quiagen HiPerformance Algorithm as indicated in the Examples. However, there are several resources for siRNA design available on the internet which allow the search for suitable siRNAs and/or RNAi targets starting from the known sequence of CD83 (Genbank entry NM_004233). Most of them use the Quiagen HiPerformance Algorithm. They include the pages: RNAi Web (http://www.rnaiweb.com/RNAi/RNAi_Web_ Resources/RNAi_Tools_Software/Online_siRNA_Design_Tools/index.html) which provides an overview on the available online-tools; the Invitrogen page (http://rnaidesigner.invitrogen.com/rnaiexpress/setOption.do?designOption=stealth ; http://rnaidesigner.invitrogen.com/rnaiexpress/setOption.do?designOption=sirna) using the award winning BLOCK-iT^{™} RNAi Designer; and the DEQOR site (http://deqor.mpi-cbg.de/) unsing a published Online Design Algorithm (Henschel, A. et al., Nucleic Acids Res. 1:32 (2004)). They are all suitable for design of the RNAi agent of present invention.

Entering NM_004233 (CD83 3' UTR, CDS and 5' UTR) into the http://rnaidesigner.invitrogen.com/rnaiexpress/setOption.do?designOption=stealth design tool results in the following target sequences inside the CD83 gene:

| No. | start nt | target sequence | origin |
|---|---|---|---|
| 1 | 677 | CGGCTACAGAGTATCTTCCCAGATT | ORF |
| 2 | 678 | GGCTACAGAGTATCTTCCCAGATTT | ORF |
| 3 | 881 | TACGCTGAAGATGGCATCCTGTGAA | UTR3 |
| 4 | 1031 | GCGATGTATGCAGCTATCTGGTCAA | UTR3 |
| 5 | 1413 | AGCAATAAAGAAGAGTGCCACATTT | UTR3 |
| 6 | 1568 | GGTGACAAGATAGAGAGCTATTTAA | UTR3 |
| 7 | 1648 | CCACTATCTGGGTGCATGATCTTGA | UTR3 |
| 8 | 1713 | CAAGGCTGTTGCATGGGCTAATGAA | UTR3 |
| 9 | 1897 | GAAGCCTTTCCTGTAGCCTTCTGTA | UTR3 |
| 10 | 2194 | ACCTCTGTCTTGGCTTTCATGTTAT | UTR3 |

Entering only the CDS of NM_004233 into the http://rnaidesigner.invitrogen.com/ rnaiexpress/setOption.do?designOption=sirna design tool results in the following target sequences:

| | | | |
|---|---|---|---|
| 1 | 324 | GGGTCAAGTTATTGGAGGGTGGTGA | ORF |
| 2 | 325 | GGTCAAGTTATTGGAGGGTGGTGAA | ORF |
| 3 | 328 | CAAGTTATTGGAGGGTGGTGAAGAG | ORF |
| 4 | 446 | TATTCCCTGAAGATCCGAAACACTA | ORF |
| 5 | 522 | AGAGAAACCTAAGTGGCAAGGTGAT | ORF |
| 6 | 525 | GAAACCTAAGTGGCAAGGTGATCTT | ORF |
| 7 | 544 | GATCTTGAGAGTGACAGGATGCCCT | ORF |
| 8 | 677 | CGGCTACAGAGTATCTTCCCAGATT | ORF |
| 9 | 678 | GGCTACAGAGTATCTTCCCAGATTT | ORF |
| 10 | 769 | GACTCCTCACAAGACAGAACTGGTA | ORF |

Entering NM_004233 (5' UTR, CDS, 3' UTR) into the DEQOR design tool results in the following siRNA duplexes (which, of course, will contain U instead of T):

| Window Sequence | | | | |
|---|---|---|---|---|
| 316 | GGTCTCCTGGGTCAAGTTATT | (-> sense) | TGCCAGAGGACCCAGTTCAAT | (<- antisense) |
| 317 | GTCTCCTGGGTCAAGTTATTG | (-> sense) | GCCAGAGGACCCAGTTCAATA | (<- antisense) |
| 328 | CAAGTTATTGGAGGGTGGTGA | (-> sense) | CAGTTCAATAACCTCCCACCA | (<- antisense) |
| 331 | GTTATTGGAGGGTGGTGAAGA | (-> sense) | TTCAATAACCTCCCACCACTT | (<- antisense) |
| 386 | GGACAGCACTATCATCAGAAG | (-> sense) | CCCCTGTCGTGATAGTAGTCT | (<- antisense) |
| 387 | GACAGCACTATCATCAGAAGG | (-> sense) | CCCTGTCGTGATAGTAGTCTT | (<- antisense) |
| 445 | CTATTCCCTGAAGATCCGAAA | (-> sense) | GGGATAAGGGACTTCTAGGCT | (<- antisense) |
| 451 | CCTGAAGATCCGAAACACTAC | (-> sense) | AGGGACTTCTAGGCTTTGTGA | (<- antisense) |
| 452 | CTGAAGATCCGAAACACTACC | (-> sense) | GGGACTTCTAGGCTTTGTGAT | (<- antisense) |
| 515 | GATGGGCAGAGAAACCTAAGT | (-> sense) | GCCTACCCGTCTCTTTGGATT | (<- antisense) |
| 521 | CAGAGAAACCTAAGTGGCAAG | (-> sense) | CCGTCTCTTTGGATTCACCGT | (<- antisense) |
| 525 | GAAACCTAAGTGGCAAGGTGA | (-> sense) | CTCTTTGGATTCACCGTTCCA | (<- antisense) |
| 537 | GCAAGGTGATCTTGAGAGTGA | (-> sense) | ACCGTTCCACTAGAACTCTCA | (<- antisense) |
| 567 | CTGCACAGCGTAAAGAAGAGA | (-> sense) | GGGACGTGTCGCATTTCTTCT | (<- antisense) |
| 764 | CTAGTGACTCCTCACAAGACA | (-> sense) | CCGATCACTGAGGAGTGTTCT | (<- antisense) |
| 778 | CAAGACAGAACTGGTATGAGC | (-> sense) | GTGTTCTGTCTTGACCATACT | (<- antisense) |
| 785 | GAACTGGTATGAGCAGGATTT | (-> sense) | GTCTTGACCATACTCGTCCTA | (<- antisense) |
| 798 | CAGGATTTCTGCAGGTTCTTC | (-> sense) | TCGTCCTAAAGACGTCCAAGA | (<- antisense) |
| 801 | GATTTCTGCAGGTTCTTCTTC | (-> sense) | TCCTAAAGACGTCCAAGAAGA | (<- antisense) |
| 811 | GGTTCTTCTTCCTGAAGCTGA | (-> sense) | GTCCAAGAAGAAGGACTTCGA | (<- antisense) |
| 853 | GTTACACTGGAGGAGAGAAGA | (-> sense) | GACAATGTGACCTCCTCTCTT | (<- antisense) |
| 857 | CACTGGAGGAGAGAAGAATGA | (-> sense) | ATGTGACCTCCTCTCTTCTTA | (<- antisense) |
| 887 | GAAGATGGCATCCTGTGAAGT | (-> sense) | GACTTCTACCGTAGGACACTT | (<- antisense) |
| 1036 | GTATGCAGCTATCTGGTCAAC | (-> sense) | TACATACGTCGATAGACCAGT | (<- antisense) |
| 1111 | GGGTCTTGGGAAATATGAATG | (-> sense) | TTCCCAGAACCCTTTATACTT | (<- antisense) |
| 1112 | GGTCTTGGGAAATATGAATGC | (-> sense) | TCCCAGAACCCTTTATACTTA | (<- antisense) |
| 1239 | CGTCTGGGAGAAATTGACAGA | (-> sense) | ATGCAGACCCTCTTTAACTGT | (<- antisense) |
| 1242 | CTGGGAGAAATTGACAGATCA | (-> sense) | CAGACCCTCTTTAACTGTCTA | (<- antisense) |
| 1244 | GGGAGAAATTGACAGATCAAG | (-> sense) | GACCCTCTTTAACTGTCTAGT | (<- antisense) |
| 1245 | GGAGAAATTGACAGATCAAGC | (-> sense) | ACCCTCTTTAACTGTCTAGTT | (<- antisense) |
| 1248 | GAAATTGACAGATCAAGCTGT | (-> sense) | CTCTTTAACTGTCTAGTTCGA | (<- antisense) |
| 1254 | GACAGATCAAGCTGTGAGACA | (-> sense) | AACTGTCTAGTTCGACACTCT | (<- antisense) |
| 1256 | CAGATCAAGCTGTGAGACAGT | (-> sense) | CTGTCTAGTTCGACACTCTGT | (<- antisense) |
| 1264 | GCTGTGAGACAGTGGGAAATA | (-> sense) | TTCGACACTCTGTCACCCTTT | (<- antisense) |
| 1265 | CTGTGAGACAGTGGGAAATAT | (-> sense) | TCGACACTCTGTCACCCTTTA | (<- antisense) |
| 1267 | GTGAGACAGTGGGAAATATTT | (-> sense) | GACACTCTGTCACCCTTTATA | (<- antisense) |
| 1269 | GAGACAGTGGGAAATATTTAG | (-> sense) | CACTCTGTCACCCTTTATAAA | (<- antisense) |
| 1273 | CAGTGGGAAATATTTAGCAAA | (-> sense) | CTGTCACCCTTTATAAATCGT | (<- antisense) |
| 1277 | GGGAAATATTTAGCAAATAAT | (-> sense) | CACCCTTTATAAATCGTTTAT | (<- antisense) |
| 1278 | GGAAATATTTAGCAAATAATT | (-> sense) | ACCCTTTATAAATCGTTTATT | (<- antisense) |
| 1279 | GAAATATTTAGCAAATAATTT | (-> sense) | CCCTTTATAAATCGTTTATTA | (<- antisense) |
| 1289 | GCAAATAATTTCCTGGTGTGA | (-> sense) | ATCGTTTATTAAAGGACCACA | (<- antisense) |
| 1317 | GCTATTACTAAGGAGTAATCT | (-> sense) | GACGATAATGATTCCTCATTA | (<- antisense) |
| 1331 | GTAATCTGTGTACAAAGAAAT | (-> sense) | CTCATTAGACACATGTTTCTT | (<- antisense) |
| 1395 | GAAAGACATCCATAAAGAAGC | (-> sense) | CCCTTTCTGTAGGTATTTCTT | (<- antisense) |
| 1658 | GGTGCATGATCTTGAGCAAGT | (-> sense) | ACCCACGTACTAGAACTCGTT | (<- antisense) |
| 1661 | GCATGATCTTGAGCAAGTTCC | (-> sense) | CACGTACTAGAACTCGTTCAA | (<- antisense) |
| 1686 | GGTGTCTGCTTTCTCCATTGT | (-> sense) | GACCACAGACGAAAGAGGTAA | (<- antisense) |
| 1727 | GGGCTAATGAAGATCATATAC | (-> sense) | TACCCGATTACTTCTAGTATA | (<- antisense) |
| 1728 | GGCTAATGAAGATCATATACG | (-> sense) | ACCCGATTACTTCTAGTATAT | (<- antisense) |
| 1774 | GCACTATACAGATTCGAAACT | (-> sense) | TTCGTGATATGTCTAAGCTTT | (<- antisense) |
| 1788 | CGAAACTCCATTGAGTCATTA | (-> sense) | AAGCTTTGAGGTAACTCAGTA | (<- antisense) |
| 1789 | GAAACTCCATTGAGTCATTAT | (-> sense) | AGCTTTGAGGTAACTCAGTAA | (<- antisense) |
| 1795 | CCATTGAGTCATTATCCTTGC | (-> sense) | GAGGTAACTCAGTAATAGGAA | (<- antisense) |
| 1800 | GAGTCATTATCCTTGCTATGA | (-> sense) | AACTCAGTAATAGGAACGATA | (<- antisense) |
| 1802 | GTCATTATCCTTGCTATGATG | (-> sense) | CTCAGTAATAGGAACGATACT | (<- antisense) |
| 1811 | CTTGCTATGATGATGGTGTTT | (-> sense) | AGGAACGATACTACTACCACA | (<- antisense) |
| 1836 | GATGAGAGGGTGCTATCCATT | (-> sense) | CCCTACTCTCCCACGATAGGT | (<- antisense) |
| 1839 | GAGAGGGTGCTATCCATTTCT | (-> sense) | TACTCTCCCACGATAGGTAAA | (<- antisense) |
| 1841 | GAGGGTGCTATCCATTTCTCA | (-> sense) | CTCTCCCACGATAGGTAAAGA | (<- antisense) |
| 1843 | GGGTGCTATCCATTTCTCATG | (-> sense) | CTCCCACGATAGGTAAAGAGT | (<- antisense) |
| 1844 | GGTGCTATCCATTTCTCATGT | (-> sense) | TCCCACGATAGGTAAAGAGTA | (<- antisense) |
| 1868 | CCATTGTTTGAAACAAAGAAG | (-> sense) | AAGGTAACAAACTTTGTTTCT | (<- antisense) |
| 1869 | CATTGTTTGAAACAAAGAAGG | (-> sense) | AGGTAACAAACTTTGTTTCTT | (<- antisense) |
| 1873 | GTTTGAAACAAAGAAGGTTAC | (-> sense) | AACAAACTTTGTTTCTTCCAA | (<- antisense) |
| 1877 | GAAACAAAGAAGGTTACCAAG | (-> sense) | AACTTTGTTTCTTCCAATGGT | (<- antisense) |
| 1881 | CAAAGAAGGTTACCAAGAAGC | (-> sense) | TTGTTTCTTCCAATGGTTCTT | (<- antisense) |
| 1885 | GAAGGTTACCAAGAAGCCTTT | (-> sense) | TTCTTCCAATGGTTCTTCGGA | (<- antisense) |
| 1902 | CTTTCCTGTAGCCTTCTGTAG | (-> sense) | CGGAAAGGACATCGGAAGACA | (<- antisense) |
| 1906 | CCTGTAGCCTTCTGTAGGAAT | (-> sense) | AAGGACATCGGAAGACATCCT | (<- antisense) |
| 1907 | CTGTAGCCTTCTGTAGGAATT | (-> sense) | AGGACATCGGAAGACATCCTT | (<- antisense) |
| 1909 | GTAGCCTTCTGTAGGAATTCT | (-> sense) | GACATCGGAAGACATCCTTAA | (<- antisense) |
| 1958 | GGTCTGTTCTTGAAGCAGTAG | (-> sense) | TGCCAGACAAGAACTTCGTCA | (<- antisense) |
| 1959 | GTCTGTTCTTGAAGCAGTAGC | (-> sense) | GCCAGACAAGAACTTCGTCAT | (<- antisense) |
| 1963 | GTTCTTGAAGCAGTAGCCTAA | (-> sense) | GACAAGAACTTCGTCATCGGA | (<- antisense) |
| 1969 | GAAGCAGTAGCCTAACACACT | (-> sense) | AACTTCGTCATCGGATTGTGT | (<- antisense) |
| 1975 | GTAGCCTAACACACTCCAAGA | (-> sense) | GTCATCGGATTGTGTGAGGTT | (<- antisense) |
| 1978 | GCCTAACACACTCCAAGATAT | (-> sense) | ATCGGATTGTGTGAGGTTCTA | (<- antisense) |
| 1979 | CCTAACACACTCCAAGATATG | (-> sense) | TCGGATTGTGTGAGGTTCTAT | (<- antisense) |
| 1980 | CTAACACACTCCAAGATATGG | (-> sense) | CGGATTGTGTGAGGTTCTATA | (<- antisense) |
| 2026 | GACTTCACGAAGTGTTGCATG | (-> sense) | CCCTGAAGTGCTTCACAACGT | (<- antisense) |
| 2031 | CACGAAGTGTTGCATGGATGT | (-> sense) | AAGTGCTTCACAACGTACCTA | (<- antisense) |
| 2033 | CGAAGTGTTGCATGGATGTTT | (-> sense) | GTGCTTCACAACGTACCTACA | (<- antisense) |
| 2056 | GCCATTGTTGGCTTTCCCTTA | (-> sense) | ATCGGTAACAACCGAAAGGGA | (<- antisense) |
| 2057 | CCATTGTTGGCTTTCCCTTAT | (-> sense) | TCGGTAACAACCGAAAGGGAA | (<- antisense) |
| 2058 | CATTGTTGGCTTTCCCTTATC | (-> sense) | CGGTAACAACCGAAAGGGAAT | (<- antisense) |
| 2062 | GTTGGCTTTCCCTTATCAAAC | (-> sense) | AACAACCGAAAGGGAATAGTT | (<- antisense) |
| 2065 | GGCTTTCCCTTATCAAACTTG | (-> sense) | AACCGAAAGGGAATAGTTTGA | (<- antisense) |
| 2066 | GCTTTCCCTTATCAAACTTGG | (-> sense) | ACCGAAAGGGAATAGTTTGAA | (<- antisense) |
| 2073 | CTTATCAAACTTGGGCCCTTC | (-> sense) | GGGAATAGTTTGAACCCGGGA | (<- antisense) |
| 2090 | CTTCCCTTCTTGGTTTCCAAA | (-> sense) | GGGAAGGGAAGAACCAAAGGT | (<- antisense) |
| 2123 | CTTGAGTTATATGTTCACTGT | (-> sense) | ACGAACTCAATATACAAGTGA | (<- antisense) |
| 2167 | CGGATACCAAGTTGATTTAGT | (-> sense) | TTGCCTATGGTTCAACTAAAT | (<- antisense) |
| 2169 | GATACCAAGTTGATTTAGTGT | (-> sense) | GCCTATGGTTCAACTAAATCA | (<- antisense) |
| 2195 | CCTCTGTCTTGGCTTTCATGT | (-> sense) | ATGGAGACAGAACCGAAAGTA | (<- antisense) |
| 2198 | CTGTCTTGGCTTTCATGTTAT | (-> sense) | GAGACAGAACCGAAAGTACAA | (<- antisense) |
| 2200 | GTCTTGGCTTTCATGTTATTA | (-> sense) | GACAGAACCGAAAGTACAATA | (<- antisense) |
| 2379 | CCTGCACTTATACCCATGAAC | (-> sense) | TTGGACGTGAATATGGGTACT | (<- antisense) |
| 2380 | CTGCACTTATACCCATGAACT | (-> sense) | TGGACGTGAATATGGGTACTT | (<- antisense) |
| 2455 | GGATGAGTGCTAGGATGTTGT | (-> sense) | ACCCTACTCACGATCCTACAA | (<- antisense) |

In one preferred aspect of the invention, the target sequence is selected from the target sequences as listed above or is a sequence substantially homologous to any of said sequences. More preferably, the target sequence is situated inside the preferred segments of the CD83 transcript as outlined above. Even more preferred are the target sequences #1, 2, 3, and 5 as listed in Table I or a sequence substantially homologous to any of said sequences. In the utmost preferred aspect of the invention, the target sequence is either 5'-CCTGCTATTACTAAGGAGTAA-3' (nt 1314-1334 of SEQ ID NO:1) or 5'-AACGGATACCAAGTTGATTTA-3' (nt 2165-2185 of SEQ ID NO:1) or is a sequence substantially homologous to any of said sequences.

Preferably, the RNAi agent comprises a sequence which is complementary (antisense strand of the RNAi agent) or identical (sense strand of the RNAi agent) to said target sequence over at least 15 continuous nucleotides with the exception of at most two nucleotides. More preferably, said sequence comprised in the RNAi agent is 100 % complementary or identical to said target sequence.

In cases wherein the RNAi agent comprises a nucleic acid duplex, it has in an preferred aspect additional single stranded regions at the 3 '-terminus of each strand which consist of from 0 to 3, preferably from 0 to 2, more preferably 1 or 2, and most preferably 2 ribonucleotides or deoxyribonucleotides per strand. Preferably, said single stranded regions consist of deoxyribonucleotides, more preferably of thymidine (dT). The final composition of said single stranded regions influences the melting temperature of the duplex and will therefore in cases where said temperature is of relevance be modified accordingly by variation of its nucleotide composition.

It has been demonstrated that structurally symmetric (duplexes with symmetric 2-nucleotide 3'-overhangs), but primary sequence-asymmetric (different nucleotides on each end) siRNAs, from which the target-mRNA complementary antisense strand has greater propensity to be assembled into the RISC than the sense strand, show improved efficacy and specifity (Pei, Y and Tuschl, T, Nature Methods 3:670-676 (2006)). RNAi agents having such asymmetric 3'-overhangs are preferred in the context of present invention. Especially preferred are the combinations of asymmetric 3'-overhangs listen in Table I.

In a further preferred aspect, the RNAi agent comprises a ribonucleic acid duplex. In said latter duplex or in the loop region of an shRNA, from 0 to 50 % of the ribonucleotides may be replaced by deoxyribonucleotides. But a pure ribonulceic acid duplex region or loop, respectively, is preferred.

There are several oligonucleotides which are especially preferred for use in the method of embodiment (1). Namely, oligonucleotides wherein in the duplex portion of the RNAi agent the antisense strand comprises the sequence UUACUCCUUAGUAAUAGCA (nt 1 to 19 of SEQ ID NO:5), UAAAUCAACUUGGUAUCCG (nt 1 to 19 of SEQ ID NO: 13), GGCAUUUAUUGCUGAGUUA (nt 1 to 19 of SEQ ID NO:6), GGAUGGGCAGAGAAACCUA (nt 1 to 19 of SEQ ID NO:8) or a sequence substantially homologous to any of said sequences.

Consequently, the following oligonucleotides wherein in the duplex portion of the RNAi agent the sense strand comprises the sequence UGCUAUUACUAAGGAGUAA (nt 1 to 19 of SEQ ID NO:4), CGGAUACCAAGUUGAUUUA (nt 1 to 19 of SEQ ID NO:12), UAACUCAGCAAUAAAUGCC (nt 1 to 19 of SEQ ID NO:7), UAGGUUUCUCUGCCCAUCC (nt 1 to 19 of SEQ ID NO:9) or a sequence substantially homologous to any of said sequences, are also especially preferred for use in the method of embodiment (1).

Even more preferred are oligonucleotides wherein in the duplex portion of the RNAi agent
(i) the sense strand comprises the sequence UGCUAUUACUAAGGAGUAA (nt 1 to 19 of SEQ ID NO:4), and the antisense strand comprises the sequence UUACUCCUUAGUAAUAGCA (nt 1 to 19 of SEQ ID NO:5); and/or
(ii) the sense strand comprises the sequence CGGAUACCAAGUUGAUUUA (nt 1 to 19 of SEQ ID NO:12), and the antisense strand comprises the sequence UAAAUCAACUUGGUAUCCG (nt 1 to 19 of SEQ ID NO:13).

Furthermore, oligonucleotides which are isolated siRNAs consisting of the sense and antisense strands of duplexes #1, 2, 3 or 5 as listed in Table I are preferred. More preferred are isolated siRNAs consisting of the sense strand r(UGCUAUUACUAAGGAGUAA)dTdT (SEQ ID NO:4) and the antisense strand r(UUACUCCUUAGUAAUAGCA)dGdG (SEQ ID NO:5) ("siRNA duplex #1"), or of the sense strand r(CGGAUACCAAGUUGAUUUA)dTdT (SEQ ID NO:12) and the antisense strand r(UAAAUCAACUUGGUAUCCG)dTdT (SEQ ID NO:13) ("siRNA duplex #5"). The siRNA duplex #1 was the one out of a group of five siRNA duplexes - all directed against the CD83 message - that led to the most significant reduction of the total level of cellular CD83 mRNA. It was identified by Real-Time Polymerase-Chain-Reaction (RT-PCR).

The method of embodiment (1) may be performed *in vivo* or ex *vivo,* but performance *ex vivo* is preferred.

The starting DC population for the transformation in embodiment (1) may be any population of DCs isolated or developed by methods known in the art (US 6,004,807; WO 03/012081; WO 02/16560; EP 0922758; EP 0633929; Berger, T.G. et al., J. Immunol. Methods 2002, 268(2):131-140; Caux, C. et al., J. Immunol. 1995, 155(11) :5427-5435). It may be present in or isolated directly from natural sources like blood or tissue, or may be produced *in vitro,* e.g. by cell culture. Preferably, it is a population which was developed from PBMCs in cell culture (see Examples), from CD34+ hematopoetic stem cells (US 6,004,807), or which was directly isolated from blood. The starting DC population may also be a mixed DC culture or a subpopulation thereof. Said mixed cell culture comprises or preferably consists of DCs in different maturation stages including mDCs and iDCs. It can be divided into subpopulations by any suitable method like FACS. The use of iDCs as starting DC population is most preferred.

The starting DC population consists preferably of mammalian DCs, most notably of human DCs.

The transformation of the starting DC population in embodiment (1) may be performed by any transformation method suitable for delivery of the RNAi agent into the DCs. For example, the compositions may be conveniently administered by microinjection, by microparticle bombardment, by soaking the DC in a solution of the RNAi agent, by electroporation of cell membranes in the presence of the RNAi agent, by liposome-mediated delivery of the RNAi agent and transfection mediated by chemicals such as calcium phosphate, viral infection, transformation, and the like. In the case of a cell culture or tissue explant, the cells are conveniently incubated in a solution containing the RNAi agent or the RNAi agent is introduced into the cell by lipid-mediated transfection.

Direct delivery of the RNAi agent without the necessity of transcription of a vector or precursor in order to produce the active RNAi agent after transformation is preferred. As physical treatment of the cells induces intracellular expression/accumulation of CD83 and should therefore be avoided or at least dramatically reduced in order to obtain low expressing iDC, transformation by electroporation is especially preferred.

The RNAi agent is typically administered in an amount that allows delivery of at least one copy of the RNAi agent per target cell. The amount administered to a cell, tissue, or organism depends on the nature of the cell, tissue, or organism, and the nature of the RNAi agent, and can readily be optimized to obtain the desired level of CD83 expression inhibition.

Furthermore, the time point of application of the RNAi agent during DC differentiation and maturation is critical for an efficient knockdown of CD83 expression using the method of embodiment (1):

Target cells of the RNAi may be any kind of DCs, but iDCs are preferred. This is due to the fact that CD83 is very stable in its membrane-bound state. Consequently, in order to achieve an efficient knockdown of this protein, DCs that do not yet express large amounts of CD83 represent the most appropriate target (compare example 3). Thus, the CD83 expression reduction by RNAi in mature DCs may occur quite slowly. On the other hand, treatment of iDCs with CD83 siRNA results in efficient, specific and long-lasting reduction of CD83 surface expression on the treated DCs. iDC populations that were siRNA electroporated prior to maturation (figure 3A, right column) showed dramatically reduced numbers of CD83 positive cells compared with untreated and mock-electroporated cells. The most reasonable target to knockdown CD83 efficiently is therefore a starting population of iDCs.

The efficiency of siRNA mediated gene knockdown is strictly dependent on the stability of the target protein. Although efficient silencing has been achieved on mRNA level, cells might still contain large amounts of the respective protein, due to its high stability. Thus, DCs that do not yet express large amounts of CD83 represent the most appropriate target for the transformation according to embodiment (1). Freshly prepared iDCs contain almost no CD83 and therefore represent the most favorable target for CD83 knockdown. iDCs can be prepared by the method described in example 1(A), more generally from blood or by development from blood components, preferably by development from PBMCs (compare example 1) or CD34+ hematopoetic stem cells (as described in US 6,004,807), but also by one of the other methods described in WO 03/012081; WO 02/16560; EP 0922758; EP 0633929; Berger, T.G. et al., J. Immunol. Methods 2002, 268(2):131-140; or Caux, C. et al., J. Immunol. 1995, 155(11) :5427-5435.

Furthermore, it has been found that mechanical treatment of the cells (which usually leads to cell damage by shear forces) obviously induces intracellular expression/accumulation of CD83 (compare Example 3) and should therefore preferably be avoided or at least dramatically reduced in order to obtain low expressing iDC as a starting population.

When iDCs are used as starting DC population for the transformation in embodiment (1), the method preferably comprises a further step, namely a maturation subsequently to the transformation (embodiment (3)). There is a vast and still increasing number of maturation methods known in the art (reviewed *inter alia* in: Steinman R. et al., J Invest Dermatol. 1995 Jul;105(1 Suppl):2S-7S; Jonuleit H. et al., Arch Dermatol Res. 1996 Dec;289(1):1-8; Cella M. et al, Curr Opin Immunol. 1997 Feb;9(1):10-6; Aiba S. et al., Tohoku J Exp Med. 1998 Mar;184(3):159-72; Rescigno M et al., J Clin Immunol. 2000 May;20(3):161-6; Flores-Romo L., Immunology. 2001 Mar;102(3):255-62; Quah BJ, and O'Neill HC, J Cell Mol Med. 2005 Jul-Sep;9(3):643-54; Sheng KC et al., Curr Med Chem. 2005;12(15):1783-800), and they are all suitable for performance of the maturation step according to present invention. However, the maturation in embodiment (3) is preferably achieved by addition of a maturation cocktail comprising one or more maturation factors to the cell culture containing the transformed cells. This is due to the easy handling of the maturation cocktail and of its components. A variety of maturation factors are available. Preferred maturation factors and combination of maturation factors are the following: TNF-α, IL-1, IL-6, prostaglandin E2 (PGE2) alone or in various combinations. The maturation cocktail may optionally contain prostaglandins (monocyte conditioned medium: Thurner B. et al., J. Immunol. Methods 223(1):1-15 (1999); defined cocktail: Jonuleit H. et al., Eur. J. Immunol. 27(12):3135-42 (1997)), various TLR ligands (Napolitani G. et al., Nat. Immunol. 6(8):769-76 (2005); Gautier G. et al., J. Exp. Med. 201(9):1435-46 (2005)); CD40 crosslinking by CD40 ligand and optionally containing IFN-γ (Caux C. et al., J. Exp. Med. 180(4):1263-72 (1994); Koya R.C. et al., J. Immunother. 26(5):451-60 (2003)), type I interferon (Gautier G. et al., J. Exp. Med. 201(9): 1435-46 (2005)), GMCSF, IL-4 and combinations thereof.

Said maturation cocktail preferably comprises at least three, but more preferably all, of the compounds selected from the group consisting of tumor necrosis factor alpha (TNFα), prostaglandin E2 (PGE2), Interleukin-1β (IL-1β), and IL-6. In a preferred aspect, said cocktail additionally comprises one or both of granulocyte macrophage colony-stimulating factor (GMCSF) and Interleukin-4 (IL-4).

Particularly preferred is a maturation cocktail comprising TNF-α, IL-1β, IL-6, and PGE₂.

The final concentrations of said ingredients of the maturation cocktail in the cell culture medium are preferably within a range from 0.1 ng/ml to 100 µg/ml, more preferably from 1 ng/ml to 10 µg/ml per ingredient. For the particularly preferred maturing cocktail of present invention, the concentration of the maturing stimulants is from 0.1 to 100 ng/ml TNFα, from 0.1 to 10 µg/ml PGE2, from 0.1 to 100 ng/ml (from 2 to 2000 U/ml) IL-1β, from 0.8 to 800 U/ml (from 0.1 to 100 ng/ml) GMCSF, from 10 to 10000 U/ml (from 0.1 to 100 ng/ml) IL-4, and from 0.1 to 100 ng/ml (from 10 to 10000 U/ml) IL-6; most preferably it is 10 ng/ml TNFα, 1 µg/ml PGE2, 200 U/ml (10 ng/ml) IL-1β, 40 U/ml GMCSF, 1000 U/ml (10 ng/ml) IL-6 and 250 U/ml IL-4. The concentration stated are the final concentrations of the substances in the cell culture medium in which the DCs are cultured.

Of course, the ingredients of above-described maturation cocktail may also be applied to the cell culture without mixing them to a "cocktail" beforehand. The cocktail, however, is preferred, as it shortens the time needed for addition of all ingredients to the cells.

Contrary to iDCs, mDCs can be frozen and stored without losing their characteristical features and biological activity (WO 02/16560; Feuerstein, B. et al., J. Immunol. Methods 2000 245(1-2):15-29). Therefore, the method of embodiment (3) preferably further comprises freezing and storage of the mature DC resulting from the transformation and the subsequent maturation steps.

Finally, it may be advatageous to remove from the DCs resulting from the methods of embodiments (1) to (3) any cells which still express CD83 on their surface ("CDhigh" in Fig. 4) before using the DCs with reduced or no CD83 expression ("CDlow" in Fig. 4). Therefore, in one preferred aspect the method of embodiment (1) to (3) comprises such removal step. The removal may be performed by any cell sorting method, but sorting by FACS (as exemplified in Example 5) is preferred.

Present invention provides a method for treating or preventing a disease or clinical condition caused by the dysfunction or undesired function of a cellular immune response involving DCs, T-cells and/or B-cells and the mCD83 expressed on the surface of said cells. The presence of mCD83 plays a crucial role in said cellular immune response, either because mCD83 itself is mediating or even causing an undesired interaction between the immunocytes, or because its presence is the indirect cause for said interaction.

In embodiment (7), present invention provides a method for treating or preventing a disease or clinical condition caused by the dysfunction or undesired function of a cellular immune response mediated by dendritic cells. This includes the mediation via interaction of the DCs with T cells and/or B cells. The clinical condition or disease is preferably caused by a CD83 mediated interaction between mature DCs and T-cells. The therapeutic effect of embodiment (7) is based on the fact that DCs with reduced or no CD83 surface expression are unable or at least severely hampered to interact with T cells and/or B cells. Thus, the immune response triggered by this interaction is prevented.

The method of treating or prophylaxis comprises administering to a patient in need of such treatment a pharmaceutically suitable amount of the RNAi agent of embodiment (5) *("in vivo* targeting") or the dendritic cells of embodiment (6). The present invention also relates to the use of the RNAi agent of embodiment (5) or the dendritic cells of embodiment (6) for the production of a medicament for the treatment or prevention of a disease or medical condition caused by the dysfunction or undesired function of a cellular immune response mediated by dendritic cells (embodiment (8)).
The use of the RNAi agent of embodiment (5) or the dendritic cells of embodiment (6) for the prevention or treatment of diseases such as allergies, asthma, rejection of a tissue or organ transplant, autoimmune syndromes such as myasthemia gravis, multiple sclerosis, vasculitis, cronic inflammatory bowl diseases such as Morbus Crohn or colitis ulcerosa, HLA B27-associated autoimmunopathis such as Morbus Bechterew, and systemic lupus erythematosis, skin diseases such as psoriasis, rheumatoid arthritis, insulin-dependent diabetes mellitus and AIDS is preferred. The methods might be applied for the treatment or prevention of diseases such as allergies, asthma, rejection of a tissue or organ transplant, autoimmune syndromes such as myasthemia gravis, multiple sclerosis, vasculitis, cronic inflammatory bowl diseases such as Morbus Crohn or colitis ulcerosa, HLA B27-associated autoimmunopathis such as Morbus Bechterew, and systemic lupus erythematosis, skin diseases such as psoriasis, rheumatoid arthritis, insulin-dependent diabetes mellitus and AIDS.

Methods of treatment and/or prevention of medical conditions caused by dysfunction or undesired function of a cellular immune response mediated by dendritic cells comprise administering an effective amount of the DCs or the RNAi agent to a patient in need thereof. Said patient is preferably a mammal, more preferably a human.

As defined herein, the term "inhibit the interaction" is used to indicate that the reduction or complete loss of CD83 on the DC surface is capable of disrupting the interaction of dendritic cells to T cells and/or B cells *in vitro* at physiological pH and salt concentrations, preferably, at pH concentrations ranging from pH 6.0 to 8.0 and/or at salt concentrations ranging from 50 mM to 250 mM, preferably 125 mM to 175 mM. A preferred assay for determining the binding of dendritic cells to T cells is provided in WO 2004/046182 and Lechmann, M. et al. (2001) J. Exp. Med. 194:1813-1821.

The dendritic cells of embodiment (6) generally exhibit a reduced or no CD83 surface expression and contain or were transformed with the RNAi agent of embodiment (5). Preferably, they contain the RNAi agent. They are suitable for the treatment or prevention of a disease or medical condition caused by the dysfunction or undesired function of a cellular immune response involving dendritic cells, T cells and/or B cells. Such conditions include paralysis, especially paralysis associated with progressive multiple sclerosis, allergies, asthma, rejection of a tissue or organ transplant (graft rejection), autoimmune syndromes such as myasthemia gravis, multiple sclerosis, vasculitis, chronic inflammatory bowel diseases such as Morbus Crohn or colitis ulcerosa, HLA B27-associated autoimmunopathies such as Morbus Bechterew, and systemic lupus erythematosis, skin diseases such as psoriasis, rheumatoid arthritis, insulin-dependent diabetes mellitus and AIDS. Of these, the dendritic cells are especially suitable for the treatment or prevention of graft rejection and autoimmune diseases.

The dendritic cells of embodiment (6) are furthermore especially suitable for autologous donation, i.e. the donation of a patient's own DCs to said patient after RNAi transformation of the DCs *in vitro.*

Finally, the comparison of siRNA treated DCs with untreated DCs in their capacity to activate T-cells in a mixed leukocyte reaction (MLR) shows a significant reduction of the siRNA treated cells in their capability to induce T-cell proferation. This result indicates that CD83 has an enhancing effect on the T-cell stimulatory capacity of mDCs. More precisely, the membrane bound form of CD83 acts as an enhancer during the stimulation of T-cell by DCs. This was shown via a specific and efficient knockdown of CD83 by siRNA electroporation (compare example 5). Thus, the embodiment (4) is one embodiment of present invention.

In one preferred aspect of present invention, the method of embodiment (7) is performed by isolation of DCs from a patient, subsequent specific knockdown of CD83 and re-transfer of the CD83-silenced cells into the patient (autologous donation). In another preferred aspect, the method is performed by a known *in vivo* application method for RNAi agents as described in the art, especially in Karagiannis, T.C. and El-Osta, A., Cancer Biol. Ther. 2004, 3(11): 1069-74; Lu, P.Y et al., Adv. Genet. 2005, 54:117-42; Aigner, A. J., Biotechnol. 2006, 124(1):12-25; Dykxhoorn, D.M. and Lieberman, J., Annu. Rev. Med. 56:401-423 (2005); Dykxhoorn, D.M. and Lieberman, J., Cell 126:231-235 (2006); and Dykxhoorn, D.M. and Lieberman, J., Annu. Rev. Biomed. Eng. 8:377-402 (2006).

The medicament of embodiment (8) can be used to treat or prevent undesirable response to foreign antigens and therewith allergies and asthma or similar conditions.
Other disorders, diseases and syndromes that can be treated or prevented by the use of the medicament of embodiment (8) include autoimmune syndromes such as myasthemia gravis, multiple sclerosis, vasculitis, chronic inflammatory bowel diseases such as Morbus Crohn or colitis ulcerosa, HLA B27-associated autoimmunopathies such as Morbus Bechterew, and systemic lupus erythematosis, skin diseases such as psoriasis, rheumatoid arthritis, insulin-dependent diabetes mellitus and AIDS.

For therapeutic or prophylactic use, the compounds of the present invention alone, or in combination with other immune modulatory compounds, e.g. tolerance inducing antigens, are administered to a subject, preferably a mammal, more preferably a human patient, for treatment or prevention in a manner appropriate for the medical indication. Transcutan, intracutan, subcutan and/or systemic administration may be chosen for the delivery of the medicament.
The production of pharmaceutical compositions with an amount of one or more compounds according to the invention and/or their use in the application according to the invention occurs in the customary manner by means of common pharmaceutical technology methods. For this, the compounds according to the invention are processed together with suitable, pharmaceutically acceptable adjuvants and/or carriers to medicinal forms suitable for the various indications and types of application. Thereby, the medicaments can be produced in such a manner that the respective desired release rate is obtained, for example a quick flooding and/or a sustained or depot effect.
The medicament of embodiment (8) may additionally comprise a pharmaceutically acceptable carrier. A "pharmaceutically acceptable carrier" according to the present invention is any carrier used in pharmaceutical compositions including a lipid, in particular a liposome, and nanoparticles. The medicament may additionally contain components that enhance uptake of the RNAi agent by the cell, stabilize the annealed strands of a nucleic acid duplex, or otherwise increase inhibition of the target gene.

The RNAi agents for use in the medicament can be administered in conjunction with agents that increase cell membrane permeability and/or cellular uptake of nucleic acids. Examples of these agents are polyamines as described for example by Antony, T. et al. (1999) Biochemistry 38:10775-10784; branched polyamines as described for example by Escriou, V. et al (1998) Biochem. Biophys. Acta 1368(2):276- 288; polyaminolipids as described for example by Guy-Caffey, J.K. et al. (1995) J. Biol. Chem. 270(52): 31391-31396; DOTMA as desribed by Felgner, P.L. et al. (1987) PNAS USA 84(21): 7413-7417 and cationic porphyrins as described for example by Benimetskaya, L. et al. (1998) NAR 26(23):5310-5317.

The medicament may be administered to a subject in need of clinical treatment by any way which allows for a distribution of the agent within the organism which may or may not be locally limited, especially by any mode of siRNA administration and delivery known in the art (Xie, F.Y. et al., Drug Discov. Today 11:67-73 (2006); Gilmore, I.R. et al., Curr. Drug. Deliv. 3:147-5 (2006); Dykxhoorn, D.M. and Lieberman, J., Annu. Rev. Biomed. Eng. 8:377-402 (2006)). Such an administration may for instance be an intratracheal, oral, topical, parenteral (including subcutaneous, intramuscular and intravenous), vaginal, rectal into the intestine, e.g. using a suppository, intranasal, ophthalmic, or intraperitoneal administration, injection or perfusion into a cavity or interstitial space of an organism, or be accomplished by absorption through the skin or by any other known method suitable for systemic or local administration. In addition, the composition may be administered via an implantable extended release device. Methods for oral introduction include direct mixing of the composition with food of the organism.
The preferred way of administration is parenteral (especially subcutaneous, intramuscular or intravenous) or intratracheal, more preferably intravenous.
Preparations for parenteral use, to which injections and infusions belong, are among the most important systemically employed medicaments for the above mentioned indications.
Preferably, injections are prepared either in the form of vials or also as so-called ready-to-use injection preparations, for example as ready-to-use syringes or single use syringes in addition to perforation bottles for multiple withdrawals. Administration of the injection preparations can occur in the form of subcutaneous (s.c.), intramuscular (i.m.), intravenous (i.v.), internodal (i.n.) or intracutaneous (i.e.) application. The respective suitable injection forms can especially be produced as solutions, crystal suspensions, nanoparticular or colloid-disperse systems, such as for example, hydrosols.
The injectable formulations can also be produced as concentrates which can be adjusted with aqueous isotonic dilution agents to the desired dosage of the compounds of the invention. Furthermore, they can also be produced as powders, such as for example lyophilisates, which are then preferably dissolved or dispersed immediately before application with suitable diluents. The infusions can also be formulated in the form of isotonic solutions, fat emulsions, liposome formulations, microemulsions and liquids based on mixed micells, for example, based on phospholipids. As with injection preparations, infusion formulations can also be prepared in the form of concentrates to dilute. The injectable formulations can also be applied in the form of continuous infusions as in stationary as well as in outpatient therapy, for example in the form of mini-pumps.

Albumin, plasma expanders, surface active compounds, organic solvents, pH influencing compounds, complex forming compounds or polymeric compounds can be added to the parenteral medicinal forms with the aim of decreasing the adsorption of the compounds of the present invention to materials such as injection instruments or packaging materials, for example plastic or glass.

If cells (especially DCs) transformed with the RNAi agent are administered to the subject, autologous donation is preferred.

Target cells of the RNAi may be not only any kind of DCs, but also CD83-positive T cells (Wolenski, M. et al., Scand. J. Immunol. 58:306-311 (2003)), B cells and macrophages (Cao, W. et al., Biochem. J. 385:85-93 (2005)). However, any kind of DCs are preferred, as they are the only kind of cells which do stably express CD83 on their surface (Cao, W. et al., Biochem. J. 385:85-93 (2005)).

Nevertheless, in embodiment (10) present invention provides a method for the specific reduction of CD83 surface expression by T-cells or B-cells, comprising the transfection of a starting T-cell or B-cell population with a RNAi agent which is (i) targeted to a transcript encoding the membrane associated CD83 (mCD83) and (ii) capable of selectively reducing or inhibiting the mCD83 expression on the cell surface. This method differs from the method of embodiment (1) only in the kind of transfected cells. Thus, the RNAi agent and its target sequence in embodiment (10) are the same as described above for embodiment (1). And furthermore, the method of embodiment (10) is preferably performed as described above, namely by using electroporation for the transfection step and/or by performing the method *ex vivo.*

In embodiment (10), the starting T-cell or B-cell population consists either of inactivated or activated T-cells or B-cells, or of a mixture of inactivated and activated cells. A starting population consisting of inactivated cells is preferred, as - similar to iDCs - these cells to not express mCD83. This is due to the fact that generally mCD83 expression in T- oder B-cells only takes place after activation of the cells. Therefore, it is preferred that the method of embodiment (10) is performed in a similar manner as the methods of embodiments (2) and (3) described above for DCs. The following exchanges in the method of embodiments (2) and (3) lead to this preferred aspect of embodiment (10): inactivated T- or B-cells are used instead of the iDCs, an activation step is performed instead of the maturation step, and T-cell or, respectively, B-cell activating factors are used instead of the maturation factors. Such activating factors may be any known T- or B-cell activating factors.

By using inactivated T- or B-cells and performing an activation step, an antigen specific anergy of the T- or B-cells may be achieved. Furthermore, this may result in B-cells whose antibody production is reduced or even completely suppressed.

The cells of embodiment (12) may be used as ingredient for a medicament as outlined above for the DCs of present invention. The cells are suitable for the treatment of T-cell or B-cell mediated diseases. Preferably, they are used for the treatment of the diseases listed above for the medicament of embodiment (8), more preferably for the treatment of allergies and asthma. The method of treatment using the cells of embodiment (12) can be performed in the same manner and with the same means as outlined above for the method of treatment using the DCs of present invention.

The medicament of embodiment (13) comprising the cells of embodiment (12) has the same properties as outlined above for the medicament comprising DCs or an RNAi agent.

The method of embodiment (13) is preferably performed as described for the method (7) as outlined above. WO 2004/046182 describes some of the CD83 mediated interactions wherein T-cells and/or B-cells may participate and whose dysfunction may lead to diseases. These are the preferred fields of application for the method of embodiment (13).

Present invention is based on the finding that proper upregulation of CD83 during DC maturation can be efficiently prevented by the use of siRNA electroporation into iDCs without interfering with other molecules. In a recent publication the inventors could show that the delivery of siRNA by electroporation does not influence DCs' biology and is therefore a suitable method to modulate DCs without the use of chemical transfection reagents (31). Specific knockdown of a gene without directly influencing any other mRNA is a typical and outstanding attribute of siRNA and has been intensively investigated in detail by numerous researchers (for examples see 28,40,41). However, in this respect some interesting observations have to be considered: Cella and colleagues presented results where iDCs could be activated by double stranded RNA (42). Furthermore, recently two other groups provided evidence that under certain circumstances siRNAs are able to initiate DC-maturation (43,44). This is not a general effect of siRNA, but is depending on the sequence of the duplex and has to be considered as an additional biological activity of siRNA. For this reason, they termed this immunostimulatory function as isRNA (immunostimulatory siRNA; 43). In contrast thereto, no negative side effects of the siRNA duplex #1 used in present invention were detected. Rather, expression of CD80 and CD86 comparable to the expression in untransformed DCs was achieved (see figure 1B and figure 3).

Further, simultaneous intra- and extracellular FACS staining showed that freshly prepared immature DCs are the most suitable target for the application of siRNA molecules directed against CD83 as they do not express large amounts of CD83 (see figure 2B). This observation is in contrast to data reported by Klein and co-workers. In their experiments, they showed by Western blot analyses that iDCs already contain a pool of pre-formed CD83, and speculated that upon maturation, the CD83 is transported from this intracellular reservoir to the surface, where it is expressed fully glycosylated as the membrane bound form (45). However, from the data obtained in developing present invention it is concludable that probably mechanical stress during the treatment of the immature DCs is most likely the reason for intracellular accumulation.

Little is known about the exact function of human CD83; whether a corresponding ligand for this molecule exists on DCs and/or T-cells is still controversial (16,17,46). Nevertheless, CD83 is always put into the spotlight when trying to identify factors that induce or promote generation of T-cell immunity: recently Hirano and colleagues reported that a CD83 binding molecule is induced on both CD4⁺ and

CD8⁺ human T-cells by CD28-mediated co-stimulation. Furthermore, they were able to show that CD83 delivers a signal that enhances the *in vitro* generation of cytotoxic T-lymphocytes (CTLs) (46). Supporting data to the observation made by Fujimoto and co-workers (26) came from Garcia-Martinez et al. who found that CD4⁺ T-cell development is substantially reduced in mice that bear a mutated form of the CD83 gene (47).

A further aim of present invention was to provide further information of the function of surface associated CD83 during the activation of T-cell proliferation. A population of mature but CD83low DCs showed a significant reduction in the ability to prime naïve allogenic T-cells (see figure 4C). It is noteworthy that this observation fits perfectly to data achieved by other studies. Zinser and colleagues (38) as well as Kruse and co-workers (18) were able to block CD83 surface expression and in consequence found a reduced capability of DCs to activate T-cells that corresponded very well with the results in present invention. However, in contrast to these studies, the method of present invention is a valuable tool to reduce specifically CD83 surface expression without interfering with other molecules.

Dudziak et al. recently reported that during the development of DCs different CD83 proteins are produced by alternative splicing. The longest of these transcripts codes for the membrane bound form, whereas the smaller transcripts are splice variants of full length CD83, coding for putative sCD83 proteins (22). This might be a first hint that sCD83 is not necessarily produced exclusively by shedding of mCD83 (12). However, they also found that the stimulation of PBMCs leads to a strong upregulation of the full-length CD83 transcript and to a strong down-regulation of two of the three smaller transcripts. In iDCs almost none of the smaller transcripts could be found. Hence the membrane bound form represents the optimal target to further investigate CD83 function during T-cell stimulation.

The following examples illustrate the present invention, however, they are not to be construed as limiting the invention.

### Examples

In the following examples, standard techniques of recombinant DNA technology and molecular biology were used that were described in various publications, e.g. Sambrook et al. (2001), Molecular Cloning: A Laboratory Manual, 3rd edition, Cold Spring Harbor Laboratory Press, or Ausubel et al. (1987), Current Protocols in Molecular Biology, Current Protocols in Immunology, Current Protocols in Protein Science, and Current Protocols in Cell Biology, edition as of 2002, Wiley Interscience. Unless otherwise indicated, all enzymes, antibodies, cells, reagents, devices and kits were used according to the manufacturers' instructions.

### Example 1: Material and Methods

### (A) Generation of Dendritic Cells

Peripheral blood mononuclear cells (PBMCs) were isolated from a single healthy donor by sedimentation using Lymphoprep (Nycomed Pharma AS, Oslo, Norway). 3 -5 x 10⁸ cells were seeded in 30 ml DC-medium (RPMI 1640 (BioWhittaker, Verviers, Belgium) supplemented with 1% autologous serum, 10 mM HEPES pH 7.5 (Sigma-Aldrich, Deisenhofen, Germany), 2 mM L-Glutamine (Cambrex BioScience, Verviers, Belgium), 100 U/ml Penicillin and 100 µg/ml Streptomycin (Sigma)) onto 175 m² standard tissue culture flasks (Nunc, Wiesbaden, Germany) and cultured for 1 h at 37°C and 5 % CO₂. The non-adherent fraction was then washed off with pure RPMI 1640 and discarded. The remaining adherent cells in the flask were cultured overnight at 37°C and 5 % CO₂ in 30 ml DC-medium. Subsequently, 10 ml DC medium supplemented with 800 µl 40.000 U/ml GMCSF-stock and 10 µl 1.000.000 U/ml IL-4-stock were added. Then, iDCs were generated by 48 h cultivation at 37°C and 5 % CO₂ in the resulting 40 ml DC-medium supplemented with 800 U/ml (800 µl GMSCF-stock) granulocyte-macrophage colony-stimulating factor (GMCSF, Wyeth, Pennsylvania, USA) and 250 U/ml (10 µl IL-4-stock) Interleukin-4 (IL-4, Strathmann, Hamburg, Germany). After said 48 h, 5 ml DC-medium supplemented with 400 U/ml GMCSF and 250 U/ml IL-4 were added per flask (end volume 45 ml), and the flasks were incubated again for 24-48 h at 37°C and 5 % CO₂. The non-adherent, suspended cells (i.e. iDCs) were collected by centrifugation, resuspended in fresh DC-medium and counted. Maturation was induced in new 175 m² flasks by adding a maturation cocktail containing TNFα, PGE2, IL-1β, IL-4 and IL-6 to a final concentration of 10 ng/ml tumor necrosis factor alpha (TNFα, Strathmann), 1 µg/ml prostaglandin E2 (PGE2, Sigma), 200 U/ml Interleukin-1β (IL-1β, Strathmann), 40 U/ml GMCSF, 250 U/ml Interleukin-4 (IL-4) and 1000 U/ml IL-6 to fresh DC-medium, giving 75 of the resulting supplemented medium into the flasks and adding resuspended cells to a final cell number of 0.5 x 10⁶ cells/ml. Maturation was performed at 37°C and 5 % CO₂ and completed 2 days later.

To ensure the purity of the achieved mDCs, the cell population was analyzed by FACS for contaminations with T-cells (CD3 antibody (BD, Heidelberg, Germany)), B-cells (CD19 antibody, clone S725-C1 (Caltag, Hamburg, Germany)), and NK-cells (CD56 antibody, clone MEM-188 (Immunotools, Friesoythe, Germany) together with the respective isotype-controls IgG1 and IgG2a, clone G155-178 (BD, Heidelberg)). Normally, mDC showed a purity of 85-90%.

### (B) siRNA Duplexes

The target of the siRNA was intended to be the CD83 mRNA. Its GENBANK entry number is NM_004233. However, since its first deposit it was replaced twice:
First deposit: NM_004233.1 (01.11.2000); SEQ ID NO:2
   gi|4757945|ref|NM_004233.1|CD83[4757945]
   The record has been replaced by 24475618
First replacement: NM_004233.2 (30.03.2006); SEQ ID NO:3
   gi|24475618|ref|NM_004233.2| [24475618]
   The record has been replaced by 94420687
Second replacement: NM_004233.3 (19.09.2006); SEQ ID NO:1
   Homo sapiens CD83 molecule (CD83), transcript variant 1, mRNA
   gi|94420687|ref|NM_004233.31|[94420687]

The most recent version which is represented by SEQ ID NO:1 is the version to which the present specification usually refers when discussing CD83 mRNA, if not indicated otherwise.

Design of siRNA duplexes was carried out with the Qiagen® HiPerformance siRNA Design Algorithm (technical information HiPerformance algorithm, Qiagen, 2005, wwwl.qiagen.com/literature/resources/RNAi/1030174-TI_GS_siRNA_0105.pdf; Rosenberg, A. et al., Nature Biotechnol. 2005, 23(8):995-1001). All siRNA duplexes were obtained from MWG (Martinsried, Germany) and dissolved in siRNA Suspension Buffer (Qiagen, Hilden, Germany) to a final concentration of 1 µg/µl, heated up for 1 min to 90 °C and incubated at 37 °C for 60 min. Resolved duplexes were stored in aliquots at -80 °C. For target sequences and sequences of the sense and antisense oligonucleotides see table I.

**Table I: CD83 siRNA Duplexes; SEQ ID NO:1 represents the CD83 mRNA sequence of the GENBANK entry NM_004233.3. duplex #2 was created using NM_00423.1 (SEQ ID NO:2) and is not completely present in SEQ ID NO:1; it corresponds to nt 1974 to 1992 of SEQ ID NO:2, which in turn correspond to nt 2112 to 2130 of SEQ ID NO:1. The SEQ ID NOs of the sense and antisense oligos are given in square brackets after their nucleic acid sequence.**

| duplex | Target Sequence | Sequence of Sense and Antisense Oligo | |
|---|---|---|---|
| #1 | 5'- CCTGCTATTACTAAGGAGTAA -3' (nt 1314-1334 of SEQ ID NO: 1) | | |
| | | Sense | r(UGCUAUUACUAAGGAGUAA)dTdT [4] |
| | | Antisense | r(UUACUCCUUAGUAAUAGCA)dGdG [5] |
| #2 | 5'- AAGGCATTTATTGCTGAGTTA -3' (nt 1974-1992 of SEQ iD NO:2) | | |
| | | Sense | r(GGCAUUUAUUGCUGAGUUA)dTdT [6] |
| | | Antisense | r(UAACUCAGCAAUAAAUGCC)dTdT [7] |
| #3 | 5'- CCGGATGGGCAGAGAAACCTA -3' (nt 512 to 532 of SEQ ID NO:1) | | |
| | | Sense | r(GGAUGGGCAGAGAAACCUA)dTdT [8] |
| | | Antisense | r(UAGGUUUCUCUGCCCAUCC)dGdG [9] |
| #4 | 5'- TGGGCTAATGAAGATCATATA -3' (nt 1726-1746 of SEQ ID NO:1) | | |
| | | Sense | r(GGCUAAUGAAGAUCAUAUA)dTdT [10] |
| | | Antisense | r(UAUAUGAUCUUCAUUAGCC)dCdA[11] |
| #5 | 5'- AACGGATACCAAGTTGATTTA -3' (nt 2165-2185 of SEQ ID NO: 1) | | |
| | | Sense | r(CGGAUACCAAGUUGAUUUA)dTdT[12] |
| | | Antisense | r(UAAAUCAACUUGGUAUCCG)dTdT [13] |

### (C) Electroporation of DCs

DCs were harvested, washed once with pure RPMI 1640 and once with PBS (all at room temperature). The cells were resuspended in OptiMEM without phenol red (Invitrogen Life Technologies) at a concentration of 4 x 10⁷ cells/ml. Respective amounts of siRNA-duplexes were transferred to a 4-mm cuvette (Peqlab, Erlangen, Germany) and filled up to a final volume of 100 µl with OptiMem. 100 µl of cell suspension (containing 4 x 10⁶ cells) were added and immediately pulsed in a GenePulser II (Bio-Rad). Pulse conditions were 300 V, 150 µF, 100 Ω. Immediately after electroporation, the cells were transferred into autologous medium supplemented with the previously indicated concentrations of GM-CSF and IL-4 or in case of maturation transferred to medium containing the maturation cocktail.

### (D) RNA Isolation and Reverse-Transcription

Cells were harvested and washed with PBS. Total RNA was isolated using the RNeasy Mini Kit and QIAshredder spin columns (Qiagen, Hilden, Germany). Traces of genomic DNA were removed by DNase digestion with the RNase-free DNase set (Qiagen). Subsequently, 1 µg of each RNA was reverse transcribed into a single-stranded cDNA by using avian myeloblastosis virus reverse transcriptase as specified by the manufacturer (Promega, Heidelberg, Germany).

### (E) Real-Time-PCR and Relative Quantification

Real-Time-Polymerase-Chain-Reaction (RT-PCR) on the LightCycler^{®} (Roche Diagnostics, Mannheim, Germany) was performed in glass capillaries at a total volume of 20 µl in the presence of 2 µl of 10x reaction buffer (Taq polymerase, dNTPs, MgCl₂, SYBR Green, Roche Diagnostics), and 1 µl of cDNA. 11.25 pmol of each oligonucleotide primer were added. RT-PCR was performed with an initial denaturation step of 15 min at 95°C, followed by 50 cycles of 20 sec denaturation at 94°C, 60 sec annealing at 57°C and 30 sec elongation time at 72°C. At the end of each cycle, the fluorescence emitted by the SYBR Green was measured. After completion of the cycling process, samples were subjected to a temperature ramp (from 65°C to 95°C at 0.1 °C/sec) with continuous fluorescence monitoring for melting curve analysis. The primers used were as follows: CD80 (sense (i.e. forward): 5'-GGACATGAATATATGGCCCG (SEQ ID NO:14), antisense (i.e. reverse): 5'-CAACACACTCGTATGTGCCC (SEQ ID NO:15), as described previously (4)), CD83 (sense: 5'-CCTCCAGCTTCTGCTCCTGA (SEQ ID NO:16), antisense: 5'-TCGGAGCAAGCCACCTTCAC (SEQ ID NO:17)), CD86 (sense: 5'-TTGCAAACTCTCAAAACCAA (SEQ ID NO:18), antisense: 5'-GGAATGAACACTGTCAAATT (SEQ ID NO:19)). As reference primers β-glucuronidase specific primers (sense: 5'-CTCATTTGGAATTTTGCCGATT (SEQ ID NO:20), antisense: 5'-CCGAGTGAAGATCCCCTTTTTA (SEQ ID NO:21), as described previously (34)) were used. Relative quantification was performed by the LightCycler^{®} Software according to the manufacturers' instructions.

### (F) FACS Analysis

The cell surface phenotype was analyzed by fluorescence-activated cell sorting (FACS). The following monoclonal antibodies were used: PE-labelled CD83 (clone HB15e), together with the respective isotype controls PE-IgG1 (clone MOPC-21). For intracellular CD83 staining a FITC-labelled CD83 (clone HB15e) and an FITC-labelled IgG1 (clone MOPC-21) isotype control (all antibodies were obtained from BD Biosciences, Heidelberg, Germany) were used according to the manufacturer's instructions.

Simultaneous intra- and extracellular staining was performed with the BD Cytofix/Cytoperm Kit (BD Biosciences, Heidelberg, Germany) as described by the manufacturer.

### (G) FACS Sorting

Single cell suspensions of siRNA electroporated and matured DCs were stained with PE-conjugated anti-CD83 antibody or the respective isotype-control. CD83low population (i.e. the DCs that showed same fluorescence as the isotype-control) were separated from the CD83high population (i.e. the DCs that showed higher fluorescence than the isotype-control) by one round of cell sorting using a MoFlo^{®} cell sorter (Cytomation) and analyzed for purity by FACS afterwards.

### (H) Allogeneic Mixed Leukocyte Reaction (MLR)

T-cells (2x10⁵ per well) and DCs were cocultivated for 4 days in 200 µl RPMI, supplemented with 5% human serum from a single AB donor in 96-well cell culture dishes. Then, cells were pulsed with [³H]-thymidine (1 µCi/well; Amersham Pharmacia Biotech) for 24 h. The culture supernatants were harvested onto glass fiber filtermates using an IH-110 harvester (Inotech), and filters were counted in a 1450 microplate counter (Wallac).

### (I) Statistical Methods

If not indicated otherwise, results are shown as mean ±SD. To determine the significance of variance in the experiments, data were analyzed using the Student's t-test. Significance was accepted if p<0.01.

### Example 2: Identification of a CD83 targeted siRNA Duplex that shows specific and efficient knockdown of CD83 mRNA

In order to knockdown a cellular target gene specifically and efficiently, a functional siRNA duplex - directed against the mRNA of the respective protein - has to be identified (27-30). To find a siRNA duplex that is able to reduce the level of CD83 mRNA and thereby reduces the total amount of CD83 protein, five different duplexes where synthesized (for target-sequences and sequence of the duplexes see table I).

iDCs were electroporated with 5.0 µg of the respective siRNA duplex and incubated for 4 h in DC-media. Afterwards they were matured for 48 h and finally the total RNA was isolated, digested with DNAse I, and reverse transcribed into cDNA. Real-Time Polymerase-Chain-Reaction (RT-PCR) for CD83 was performed using a Light Cycler^{®} System (Roche, Mannheim, Germany), with β-glucuronidase mRNA as internal control. The normalized ratio of the CD83 mRNA compared to the β-glucuronidase internal control was calculated using LightCycler^{®} software. Figure 1A demonstrates the knockdown efficiency of the different CD83 duplexes. The normalized ratio of untreated cells was set as 100% (figure 1A, column 1) and compared to the normalized ratio of cells that were either electroporated without siRNA (figure 1A, column 2) or together with 5.0 µg of the respective siRNA duplex (figure 1A, columns 3-7). A significant reduction of the total amount of CD83 mRNA to 50%-60% was only detectable in case of duplex #1 and duplex #5 (p<0.01, marked by asterisks; figure 1A, column 3 and 7). Duplexes #2 and #3 (columns 4 and 5) led to a less significant effect on CD83 mRNA, whilst duplex #4 (column 6) did not lead to a significant effect on CD83 mRNA. An influence of the electroporation procedure on the expression of CD83 mRNA could be excluded by a mock-electroporation experiment where no siRNA duplex was delivered into the cells at all (figure 1A, column 2).

To proof the specificity of the siRNA mediated gene-silencing, additional RT-PCRs were performed for typical DC surface markers. iDCs were electroporated with 7.5 µg of duplex #1 and treated as described above. Figure 1B shows the relative expression levels of CD83 (column 2), CD80 (column 3), CD86 (column 4) compared to DCs electroporated without siRNA that were set as 100% (column 1). While in case of CD83 mRNA a strong and significant (p<0.01) downregulation could be observed (figure 1B, column 2), the mRNA levels for CD80 (figure 2B, column 3) and CD86 (figure 2B, column 4) were not influenced at all (p=0.59 in case of CD80; p=0.71 in case of CD86). Therefore, duplex #1 represents a suitable duplex for siRNA mediated silencing of CD83 in DCs.

### Example 3: CD83 is stable over a long time and its expression is rapidly induced upon maturation

To evaluate the stability of CD83 on the surface of fully matured DCs, cycloheximide (CHX) was used to block intracellular supply with newly synthesized proteins. CHX has been described as a powerful drug to inhibit peptide synthesis (35).

Mature DCs were either incubated for 24 h, 48 h, or 72 h in the presence or absence of 10 µg/ml CHX and were analyzed by FACS staining for extracellular CD83 expression afterwards. Figure 2A demonstrates the number of cells that were positive for extracellular CD83. In the absence of CHX (left column) after 24 h 97% of the cells expressed CD83 on their surface. The number of CD83-positive cells slightly decreases over time from 93% CD83-positive cells after 48 h to 77% CD83-positive cells after 72 h. In the presence of CHX (right column), DCs showed a reduced CD83 surface expression. However, after 72 h of incubation with CHX still 55% of the cells were CD83-positive. It is possible that the observed loss of surface CD83 is also due to the toxic effects of CHX after 72 h. Nevertheless, these results demonstrate that CD83 is very stable in its membrane-bound state. Consequently, in order to achieve an efficient knockdown of this protein, DCs that do not yet express large amounts of CD83 represent the most appropriate target.

To address this point, it was next tried to identify the population of DCs that bears the lowest amounts of both intra- and extracellular CD83. Freshly prepared iDCs (day 5) were removed from the cell culture flasks by soft shaking and centrifugation. For simultaneous intra- and extracellular staining first the extracellular CD83 was stained with a PE-labeled antibody. After fixation and permeabilization, the intracellular CD83 was detected using a FITC-labeled antibody which recognized the same epitope as the first antibody. In figure 2B (topmost panel) the intra- and extracellular levels of CD83 are shown. Only 2% of the cells are double positive for intra- and extracellular CD83. This result clearly demonstrates that iDCs contain almost no CD83.

Next, the iDCs were transferred either to DC-media without maturation cocktail (figure 2B, left column) or to DC-media containing the maturation stimuli (figure 2B, right column). After 6 h incubation time, cells were removed and again stained for both intra- and extracellular CD83. When no maturation stimuli were present, only 5% of the cells were double positive for intra- and extracellular CD83 (left column, upper panel). In strong contrast, when the maturation cocktail was present for 6 h, 89% of the cells became positive for CD83 (right column, upper panel). A final determination of CD83 after 24 h revealed an interesting phenomenon: although maturation was not induced, 25% of the cells became double positive (left column, lower panel). Thus, it seems that the soft mechanical treatment of the iDCs during transfer from the culture flasks to the DC-media in six-well plates led to an intracellular expression/accumulation of CD83, as indicated by 96% of the cells being positive in fluorescence channel 1 (left column, lower panel, upper right quadrant: 25% + lower right quadrant: 71%). In fact, already 6 h after transfer of the cells a slight accumulation of intracellular CD83 (10%) was detectable (left column, upper panel, upper right quadrant: 5% + lower right quadrant: 5%) However, this CD83 was not completely transported to the cell surface as indicated by the low percentage of cells positive for fluorescence 2 (left column, lower panel, upper left quadrant: 0% + upper right quadrant: 25%). In strong contrast, the presence of maturation stimuli led to 98% double-positive cells (right column, lower panel).

Taken together, these data show that freshly prepared iDCs contain almost no CD83 and therefore represent the most favorable target for CD83 knockdown. Furthermore, mechanical treatment of the cells obviously induces intracellular expression/accumulation of CD83 and should therefore be avoided or at least dramatically reduced in order to obtain low expressing iDC.

### Example 4: The achieved CD83 knockdown is efficient, specific, and long-lasting

As the optimal time point for electroporation and a functional duplex was identified, a further aim was insight on CD83 surface expression, i.e. the protein level. Therefore, freshly prepared iDCs (that were tested by FACS for low levels of both intra- and extracellular CD83; data not shown) were electroporated with 7.5 µg siRNA duplex #1. After 4 h maturation was induced as described in example 1(A).

Cells from populations that were not treated at all (figure 3A-C, left column), cells that were mock-electroporated (figure 3A-C, middle column) and cells that were electroporated with duplex #1 (figure 3A-C, right column) were analyzed for surface expression of CD83 (figure 3A), CD80 (figure 3B) and CD86 (figure 3C) after 24 h, 48 h or 72 h. No significant differences in surface expression of CD80 and CD86 were detected at any given condition. The cells always showed high expression of the co-stimulatory molecules, typical for mDCs. In strong contrast, populations that were siRNA electroporated prior to maturation (figure 3A, right column) showed dramatically reduced numbers of CD83 positive cells compared with untreated and mock-electroporated cells. After 48 h (the time point at which DCs are usually considered as mature), only 20% were still positive for CD83 (3A, right column, middle panel), while 81% of the untreated (3A, left column, middle panel) and 80% of the mock-electroporated population (3A, middle column, middle panel) were. It is noteworthy that even 72 h after maturation induction only 29% of the duplex #1 electroporated cells were positive for CD83 (3A, right column, lower panel). This result clearly demonstrated that the achieved knockdown was specific for the CD83 protein and is also an efficient long-lasting effect.

### Example 5: CD83low mature Dendritic Cells show reduced capacity to stimulate allogeneic T-cells

To test whether siRNA duplex #1 electroporation influences DC-mediated T-cell proliferation, their stimulatory capacity was tested in an allogeneic mixed leukocyte reaction (MLR). MLR is a model for allogenic T cell activation and graft rejection. iDCs were electroporated, incubated for 4 h in DC-media and afterwards matured for 24 h (maturation method see example 1(A)). The mDCs were then seeded together with T-cells, pulsed with [³H]-thymidine, harvested 24 h later and the filters were counted. Figure 4A demonstrates that although a specific knockdown of CD83 was observed (as checked by FACS after 24 h; data not shown), no significant differences in their capability to induce T-cell proliferation was detectable between the mock-electroporated (figure 4A; black triangles) and duplex #1 electroporated population (figure 4A; white triangles). This might be due to the fact, that the duplex #1 electroporated population still contains cells that are positive for CD83 (see figure 3A; right column). The cells that still present CD83 on their surface are sufficient enough to induce strong T-cell proliferation. In order to separate the cells with no or very low CD83 expression from those with high CD83 surface expression, FACS sorting was performed prior to the setup of the MLR.

Figure 4B shows the sorting procedure: after 24 h maturation (28 h after siRNA electroporation with duplex #1) the cells were sorted into a CD83low and CD83high population. Starting with a duplex #1 electroporated population where still 42% of the cells were positive for membrane-bound CD83 (figure 4B, upper right panel), the resulting populations were again analyzed by FACS for their CD83 surface expression. While in case of the CD83 low population only 1% of the cells were still positive for CD83 (4B, lower left panel), 96% of the CD83 high population were CD83-positive (4B, lower right panel), demonstrating efficient and successful sorting.

Finally, the MLR experiment was repeated, but this time the efficiency of the CD83high to the CD83 low population in their capacity to induce T-cell proliferation was compared. Figure 4C clearly demonstrates that the CD83 low population (white squares) has a significantly reduced ability to induce T-cell proliferation when compared with the CD83high cells (black squares). It is noteworthy that the surface expression of CD80, CD86 and MHC II in the CD83low population was identical to the surface expression of these molecules in the CD83high population (FACS data not shown). This proofs that the CD83 low cells have a "mature" phenotype, except for the expression of CD83. Taken together, these results provide evidence that membrane-bound CD83 has the function of an enhancer during DC mediated T-cell proliferation.

### References

1. Banchereau, J., and R. M. Steinman. 1998. Nature 392:245.
2. Dustin, M. L., and J. A. Cooper. 2000. Nat Immunol 1:23.
3. Lanzavecchia, A., and F. Sallusto. 2001. Cell 106:263.
4. Dilioglou, S., J. M. Cruse, and R. E. Lewis. 2003. Exp. Mol. Pathol. 75:217.
5. Pardoll, D. M. 2002. Nat Rev. Immunol 2:227.
6. Cao, W., S. H. Lee, and J. Lu. 2005. Biochem. J. 385:85.
7. Wolenski, M., S. O. Cramer, S. Ehrlich, C. Steeg, B. Fleischer, and A. von Bonin. 2003. Scandinavian Journal of Immunology 58:306.
8. Zhou, L. J., R. Schwarting, H. M. Smith, and T. F. Tedder. 1992. J. Immunol. 149:735.
9. Zhou, L. J., and T. F. Tedder. 1995. J. Immunol. 154:3821.
10. Zhou, L. J., and T. F. Tedder. 1996. Proc. Natl. Acad. Sci. U. S. A 93:2588.
11. Hock, B. D., M. Kato, J. L. McKenzie, and D. N. J. Hart. 2001. Int. Immunol. 13:959.
12. Hock, B. D., L. F. Haring, A. Steinkasserer, K. G. Taylor, W. N. Patton, and J. L. McKenzie. 2004. Leukemia Research 28:237.
13. Salio, M., M. Cella, M. Suter, and A. Lanzavecchia. 1999. Eur. J. Immunol. 29:3245.
14. Kruse, M., O. Rosorius, F. Kratzer, G. Stelz, C. Kuhnt, G. Schuler, J. Hauber, and A. Steinkasserer. 2000. J. Virol. 74:7127.
15. Senechal, B., A. M. Boruchov, J. L. Reagan, D. N. Hart, and J. W. Young. 2004. Blood 103:4207.
16. Lechmann, M., D. J. E. B. Krooshoop, D. Dudziak, E. Kremmer, C. Kuhnt, C. G. Figdor, G. Schuler, and A. Steinkasserer. 2001. J. Exp. Med. 194:1813.
17. Scholler, N., M. Hayden-Ledbetter, A. Dahlin, I. Hellstrom, K. E. Hellstrom, and J. A. Ledbetter. 2002. J Immunol 168:2599.
18. Kruse, M., O. Rosorius, F. Kratzer, D. Bevec, C. Kuhnt, A. Steinkasserer, G. Schuler, and J. Hauber. 2000. J. Exp. Med. 191:1581.
19. Bevec, D., and J. Hauber. 1997. Biol. Signals 6:124.
20. Park, M. H., E. C. Wolff, and J. E. Folk. 1993. Biofactors 4:95.
21. Zinser, E., M. Lechmann, A. Golka, M. B. Lutz, and A. Steinkasserer. 2004. J. Exp. Med. 200:345.
22. Dudziak, D., F. Nimmerjahn, G. W. Bornkamm, and G. Laux. 2005. J Immunol 174:6672.
23. Hock, B. D., J. L. O'Donnell, K. Taylor, A. Steinkasserer, J. L. McKenzie, A. G. Rothwell, and K. L. Summers. 2006. Tissue Antigens 67:57.
24. Scholler, N., M. Hayden-Ledbetter, K. E. Hellstrom, I. Hellstrom, and J. A. Ledbetter. 2001. J. Immunol. 166:3865.
25. Yang, S., Y. Yang, J. Raycraft, H. Zhang, S. Kanan, Y. Guo, Z. Ronai, I. Hellstrom, and K. E. Hellstrom. 2004. PNAS 101:4990.
26. Fujimoto, Y., L. Tu, A. S. Miller, C. Bock, M. Fujimoto, C. Doyle, D. A. Steeber, and T. F. Tedder. 2002. Cell 108:755.
27. Tuschl, T., and A. Borkhardt. 2002. Mol. Interv. 2:158.
28. Tuschl, T. 2001. Chembiochem. 2:239.
29. Elbashir, S. M., J. Harborth, W. Lendeckel, A. Yalcin, K. Weber, and T. Tuschl. 2001. Nature 411:494.
30. Elbashir, S. M., W. Lendeckel, and T. Tuschl. 2001. Genes Dev. 15:188.
31. Prechtel, A. T., N. M. Turza, A. Theodoridis, M. Kummer, and A. Steinkasserer. 2006. J. Immunol. Methods 311:139.
32. Liu, G., H. Ng, Y. Akasaki, X. Yuan, M. Ehtesham, D. Yin, K. L. Black, and J. S. Yu. 2004. Eur. J. Immunol. 34:1680.
33. Hill, J. A., T. E. Ichim, K. P. Kusznieruk, M. Li, X. Huang, X. Yan, R. Zhong, E. Cairns, D. A. Bell, and W. P. Min. 2003. J. Immunol. 171:691.
34. Lossos, I. S., D. K. Czerwinski, M. A. Wechser, and R. Levy. 2003. Leukemia 17:789.
35. Obrig, T. G., W. J. Culp, W. L. McKeehan, and B. Hardesty. 1971. J. Biol. Chem. 246:174.
36. Lutz, M. B., and G. Schuler. 2002. Trends Immunol. 23:445.
37. Hauber, I., D. Bevec, J. Heukeshoven, F. Kratzer, F. Horn, A. Choidas, T. Harrer, and J. Hauber. 2005. J. Clin. Invest 115:76*.*
38. Zinser, E., N. Turza, and A. Steinkasserer. 2004. Immunobiology 209:89.
39. Prechtel, A. T., N. M. Turza, D. J. Kobelt, J. I. Eisemann, R. S. Coffin, Y. McGrath, C. Hacker, X. Ju, M. Zenke, and A. Steinkasserer. 2005. J Gen Virol 86:1645.
40. Bantounas, I., L. A. Phylactou, and J. B. Uney. 2004. J Mol. Endocrinol. 33:545.
41. Hannon, G. J. 2002. Nature 418:244.
42. Cella, M., M. Salio, Y. Sakakibara, H. Langen, I. Julkunen, and A. Lanzavecchia. 1999. J. Exp. Med. 189:821.
43. Hornung, V., M. Guenthner-Biller, C. Bourquin, A. Ablasser, M. Schlee, S. Uematsu, A. Noronha, M. Manoharan, S. Akira, F. A. de, S. Endres, and G. Hartmann. 2005. Nat Med. 11:263.
44. Sioud, M. 2005. J. Mol. Biol. 348:1079.
45. Klein, E., S. Koch, B. Borm, J. Neumann, V. Herzog, N. Koch, and T. Bieber. 2005. Int. Immunol 17:477.
46. Hirano, N., M. O. Butler, Z. Xia, S. Ansen, M. S. von Bergwelt-Baildon, D. Neuberg, G. J. Freeman, and L. M. Nadler. 2005. Blood*.*
47. Garcia-Martinez, L. F., M. W. Appleby, K. Staehling-Hampton, D. M. Andrews, Y. Chen, M. McEuen, P. Tang, R. L. Rhinehart, S. Proll, B. Paeper, M. E. Brunkow, A. G. Grandea, III, E. D. Howard, D. E. Walker, P. Charmley, M. Jonas, S. Shaw, J. A. Latham, and F. Ramsdell. 2004. J. Immunol. 173:2995.

## Claims

1. A method for the specific reduction of CD83 surface expression by dendritic cells (DCs), comprising the transfection of a starting DC population with a RNAi agent which is (i) targeted to a transcript encoding the membrane associated CD83 (mCD83) and (ii) capable of selectively reducing or inhibiting the mCD83 expression on the DC surface.

2. The method of claim 1 wherein the RNAi agent comprises a nucleic acid duplex formed by a sense and an antisense strand, preferably a ribonucleic acid duplex, more preferably the RNAi agent is a small interfering RNA (siRNA), a short hairpin RNA (shRNA) or a precursor thereof.

3. The method of claim 2, wherein said RNAi agent is an isolated siRNA or shRNA sequence and is not provided within a plasmid or vector or by a siRNA or shRNA precursor.

4. The method of claim 2 or 3, wherein said RNAi agent is a siRNA, preferably is a siRNA which comprises from 19 to 30, yet more preferably from 19 to 27, still more preferably from 21 to 27, and most preferably 21 nucleotides per single strand.

5. The method of any one of claims 2 to 4, wherein the duplex portion of the oligonucleotide is from 17 to 28 nucleotides (nt), preferably from 17 to 25 nt, more preferably 19 nt long.

6. The method of any one of claims 1 to 5, wherein the target sequence of the RNAi agent is a segment of the CD83 transcript or a sequence substantially homologous to such segment, preferably a segment having a length from 17 to 28 nt, more preferably from 17 to 25 nt, most preferably of 19 nt.

7. The method of claim 6, wherein the target sequence is located in the segment consisting of the 3'UTR and the CDS of the CD83 transcript (nt 179 to 2478 of SEQ ID NO:1), and preferably is located in the regions represented by nt 590 to 1450 or 2000 to 2478 of SEQ ID NO:1.

8. The method of claim 7, wherein the target sequence is either 5'-CCTGCTATTACTAAGGAGTAA-3' (nt 1314-1334 of SEQ ID NO:1) or 5'-AACGGATACCAAGTTGATTTA-3' (nt 2165-2185 of SEQ ID NO:1) or a sequence substantially homologous to any of said sequences.

9. The method of claim 7 or 8, wherein the RNAi agent comprises a sequence which is complementary (antisense strand of the RNAi agent) or identical (sense strand of the RNAi agent) to said target sequence over at least 15 continuous nucleotides with the exception of at most two nucleotides, preferably is 100 % complementary or identical to said target sequence.

10. The method of any one of claims 2 to 9, wherein the RNAi agent additionally has single stranded regions at the 3'-terminus of each strand ("3'-overhangs") which consist of from 0 to 3, preferably from 0 to 2, more preferably 1 or 2, and most preferably 2 ribonucleotides or deoxyribonucleotides per strand, wherein said 3'-overhangs preferably differ from each other in their nucleotide composition.

11. The method of any one of claims 2 to 10, wherein the RNAi agent comprises a ribonucleic acid duplex and wherein from 0 to 50 % of the ribonucleotides in said duplex or in the loop region of the shRNA have been replaced by deoxyribonucleotides.

12. The method of any one of claims 2 to 11, wherein in the duplex portion of the RNAi agent the antisense strand comprises the sequence UUACUCCUUAGUAAUAGCA (nt 1 to 19 of SEQ ID NO:5), UAAAUCAACUUGGUAUCCG (nt 1 to 19 of SEQ ID NO:13), GGCAUUUAUUGCUGAGUUA (nt 1 to 19 of SEQ ID NO:6), GGAUGGGCAGAGAAACCUA (nt 1 to 19 of SEQ ID NO:8) or a sequence substantially homologous to any of said sequences, preferably
(i) the sense strand comprises the sequence UGCUAUUACUAAGGAGUAA (nt 1 to 19 of SEQ ID NO:4), and the antisense strand comprises the sequence UUACUCCUUAGUAAUAGCA (nt 1 to 19 of SEQ ID NO:5); and/or
(ii) the sense strand comprises the sequence CGGAUACCAAGUUGAUUUA (nt 1 to 19 of SEQ ID NO:12), and the antisense strand comprises the sequence UAAAUCAACUUGGUAUCCG (nt 1 to 19 of SEQ ID NO:13).

13. The method of claim 12, wherein the RNAi agent is an isolated siRNA consisting of the sense strand r(UGCUAUUACUAAGGAGUAA)dTdT (SEQ ID NO:4) and the antisense strand r(UUACUCCUUAGUAAUAGCA)dGdG (SEQ ID NO:5), or of the sense strand r(CGGAUACCAAGUUGAUUUA)dTdT (SEQ ID NO:12) and the antisense strand r(UAAAUCAACUUGGUAUCCG)dTdT (SEQ ID NO:13).

14. The method of any one of claims 1 to 13, wherein the transfection of the DCs is performed by electroporation.

15. The method of any one of claims 1 to 14, which is performed *ex vivo.*

16. The method of any one of claims 1 to 15, wherein the starting DC population consists of immature DC (iDC).

17. The method of claim 16, which further comprises a maturation step after the transfection of the iDC to obtain mature DC, preferably by addition of a maturation cocktail comprising one or more of the compounds from the group consisting of tumor necrosis factor alpha (TNFα), prostaglandin E2 (PGE2), Interleukin-1β (IL-1β), and IL-6.

18. The method of claim 17, which further comprises freezing and storage of the mature DC.

19. The method of any one of claims 1 to 18, which additionally comprises a final purification step wherein cells still expressing CD83 on their surface are removed from the transfected cell population.

20. The method of claim 19, wherein the removal step takes place after the maturation step of claim 17.

21. The method of any one of claims 1 to 20 which is suitable for reduction or inhibition of the T cell activating ability of the DCs, and for reduction or suppression of T-cell proliferation in immune response.

22. A RNAi agent which is as defined in any one of claims 1 to 13 or is a nucleic acid that comprises a template for transcription of one or more RNA molecules that hybridize or self-hybridize to form a siRNA or shRNA as defined in any one of claims 1 to 13.

23. Dendritic cells obtainable by the method as defined in any one of claims 1 to 21.

24. The dendritic cells of claim 23 which are suitable for autologous donation.

25. A method for the treatment or prevention of a disease or clinical condition which is caused by the dysfunction or undesired function of a cellular immune response mediated by DCs, said method comprising administering a therapeutically effective amount of a RNAi agent as defined in claim 22 or a dendritic cell as defined in claim 23 or 24 to said subject.

26. The method of claim 25, wherein the disease or clinical condition is caused by a CD83 mediated interaction of mature DCs with T-cells and/or B cells in a mammalian subject.

27. The method of claim 25 or 26, wherein said disease or clinical condition is selected from the group consisting of allergies, asthma, rejection of a tissue or organ transplant, autoimmune syndromes such as myasthemia gravis, multiple sclerosis, vasculitis, chronic inflammatory bowel diseases such as Morbus Crohn or colitis ulcerosa, HLA B27-associated autoimmunopathies such as Morbus Bechterew, and systemic lupus erythematosis, skin diseases such as psoriasis, rheumatoid arthritis, insulin-dependent diabetes mellitus and AIDS.

28. The method of anyone of claims 25 to 27, wherein the clinical condition is an autoimmune disease, allergic reaction, or graft rejection.

29. A medicament for the treatment of a disease or clinical condition as defined in any one of claims 25 to 28, which comprises the RNAi agent of claim 22 or the dendritic cells of claim 23 or 24.

30. A method for decreasing the immunogenicity and rejection potential of a graft, said method comprising treating the prospective recipient and/or the graft itself with a composition that reduces or suppresses CD83 expression on the surface of DC, said composition comprising an RNAi agent as defined in claim 22.

31. A method for the specific reduction of CD83 surface expression by T-cells or B-cells, comprising the transfection of a starting T-cell or B-cell population with a RNAi agent which is (i) targeted to a transcript encoding the membrane associated CD83 (mCD83) and (ii) capable of selectively reducing or inhibiting the mCD83 expression on the cell surface.

32. The method of claim 31,
(i) wherein the RNAi agent and its target sequence is defined as in any one of claims 2 to 13; and/or
(ii) which is performed as described in claims 14 and/or 15.

33. The method of claim 31 or 32, wherein the starting T-cell or B-cell population consists of inactivated T-cells or B-cells.

34. The method of claim 33, wherein the method further comprises an activation step after the transfection step.

35. T-cells or B-cells obtainable by the method as defined in any one of claims 31 to 34.

36. A method for the treatment or prevention of a disease or clinical condition which is caused by the dysfunction or undesired function of a cellular immune response mediated by T-cells and/or B-cells, said method comprising administering a therapeutically effective amount of a RNAi agent as defined in claim 22 or cells as defined in claim 35 to said subject.

37. A medicament for the treatment of a disease or clinical condition as defined in claim 36 or listed in claim 28, which comprises cells of claim 35.


SEQUENCE LISTING

<110>  Friedrich-Alexander-Universität Erlangen-Nürnberg

<120>  Method to diminish CD83 expression in monocyte-derived dendritic
       cells by RNAi

<130>  061310us

<160>  21

<170>  PatentIn version 3.3

<210>  1
<211>  2478
<212>  DNA
<213>  homo sapiens


<220>
<221>  mRNA
<222>  (1)..(2478)
<223>  CD83 mRNA, version NM_004233.3

<400>  1
gacgggggcg ccccggccta agcgggacta ggagggcgcg ccacccgctt ccgctgcccg      60

ccggggaatc ccccgggctg gcgcgcaggg aagttcccga acgcgcgggc ataaaagggc     120

agccggcgcc cgcgcgccac agctctgcag ctcgtggcag cggcgcagcg ctccagccat     180

gtcgcgcggc ctccagcttc tgctcctgag ctgcgcctac agcctggctc ccgcgacgcc     240

ggaggtgaag gtggcttgct ccgaagatgt ggacttgccc tgcaccgccc cctgggatcc     300

gcaggttccc tacacggtct cctgggtcaa gttattggag ggtggtgaag agaggatgga     360

gacaccccag gaagaccacc tcaggggaca gcactatcat cagaaggggc aaaatggttc     420

tttcgacgcc cccaatgaaa ggccctattc cctgaagatc cgaaacacta ccagctgcaa     480

ctcggggaca tacaggtgca ctctgcagga cccggatggg cagagaaacc taagtggcaa     540

ggtgatcttg agagtgacag gatgccctgc acagcgtaaa gaagagactt ttaagaaata     600

cagagcggag attgtcctgc tgctggctct ggttattttc tacttaacac tcatcatttt     660

cacttgtaag tttgcacggc tacagagtat cttcccagat ttttctaaag ctggcatgga     720

acgagctttt ctcccagtta cctccccaaa taagcattta gggctagtga ctcctcacaa     780

gacagaactg gtatgagcag gatttctgca ggttcttctt cctgaagctg aggctcaggg     840

gtgtgcctgt ctgttacact ggaggagaga agaatgagcc tacgctgaag atggcatcct     900

gtgaagtcct tcacctcact gaaaacatct ggaaggggat cccaccccat tttctgtggg     960

caggcctcga aaaccatcac atgaccacat agcatgaggc cactgctgct tctccatggc    1020

caccttttca gcgatgtatg cagctatctg gtcaacctcc tggacatttt ttcagtcata    1080

taaaagctat ggtgagatgc agctggaaaa gggtcttggg aaatatgaat gcccccagct    1140

ggcccgtgac agactcctga ggacagctgt cctcttctgc atcttgggga catctctttg 1200

aattttctgt gttttgctgt accagcccag atgttttacg tctgggagaa attgacagat 1260

caagctgtga gacagtggga aatatttagc aaataatttc ctggtgtgaa ggtcctgcta 1320

ttactaagga gtaatctgtg tacaaagaaa taacaagtcg atgaactatt ccccagcagg 1380

gtcttttcat ctgggaaaga catccataaa gaagcaataa agaagagtgc cacatttatt 1440

tttatatcta tatgtacttg tcaaagaagg tttgtgtttt tctgcttttg aaatctgtat 1500

ctgtagtgag atagcattgt gaactgacag gcagcctgga catagagagg gagaagaagt 1560

cagagagggt gacaagatag agagctattt aatggccggc tggaaatgct gggctgacgg 1620

tgcagtctgg gtgctcgccc acttgtccca ctatctgggt gcatgatctt gagcaagttc 1680

cttctggtgt ctgctttctc cattgtaaac cacaaggctg ttgcatgggc taatgaagat 1740

catatacgtg aaaattattt gaaaacatat aaagcactat acagattcga aactccattg 1800

agtcattatc cttgctatga tgatggtgtt ttggggatga gagggtgcta tccatttctc 1860

atgttttcca ttgtttgaaa caaagaaggt taccaagaag cctttcctgt agccttctgt 1920

aggaattctt ttggggaagt gaggaagcca ggtccacggt ctgttcttga agcagtagcc 1980

taacacactc caagatatgg acacacggga gccgctggca gaagggactt cacgaagtgt 2040

tgcatggatg ttttagccat tgttggcttt cccttatcaa acttgggccc ttcccttctt 2100

ggtttccaaa ggcattttat tgcttgagtt atatgttcac tgtcccccta atattaggga 2160

gtaaaacgga taccaagttg atttagtgtt tttacctctg tcttggcttt catgttatta 2220

aacgtatgca tgtgaagaaa gggtgttttt ctgttttata ttcaactcat aagactttgg 2280

gataggaaaa atgagtaatg gttactaggc ttaatacctg ggtgattaca taatctgtac 2340

aatgaacccc catgatgtaa gtttacctat gtaacaaacc tgcacttata cccatgaact 2400

taaaatgaaa gttaaaaata aaaaacatat acaaataaaa aaatcccgac tttgggatga 2460

gtgctaggat gttgtaaa 2478

<210> 2
<211> 2304
<212> DNA
<213> homo sapiens

<220>
<221> mRNA
<222> (1)..(2304)
<223> CD83 mRNA, version NM_004233.1

<400> 2
cacagctctg cagctcgtgg cagcggcgca gcgctccagc catgtcgcgc ggcctccagc 60

ttctgctcct gagctgcgcc tacagcctgg ctcccgcgac gccggaggtg aaggtggctt 120

gctccgaaga tgtggacttg ccctgcaccg ccccctggga tccgcaggtt ccctacacgg 180
tctcctgggt caagttattg gagggtggtg aagagaggat ggagacaccc caggaagacc 240
acctcagggg acagcactat catcagaagg ggcaaaatgg ttctttcgac gcccccaatg 300
aaaggcccta ttccctgaag atccgaaaca ctaccagctg caactcgggg acatacaggt 360
gcactctgca ggacccggat gggcagagaa acctaagtgg caaggtgatc ttgagagtga 420
caggatgccc tgcacagcgt aaagaagaga cttttaagaa atacagagcg gagattgtcc 480
tgctgctggc tctggttatt ttctacttaa cactcatcat tttcacttgt aagtttgcac 540
ggctacagag tatcttccca gatttttcta aagctggcat ggaacgagct tttctcccag 600
ttacctcccc aaataagcat ttagggctag tgactcctca caagacagaa ctggtatgag 660
caggatttct gcaggttctt cttcctgaag ctgaggctca ggggtgtgcc tgtctgttac 720
actggaggag agaagaatga gcctacgctg aagatggcat cctgtgaagt ccttcacctc 780
actgaaaaca tctggaaggg gatcccaccc cattttctgt gggcaggcct cgaaaaccat 840
cacatgacca catagcatga ggccactgct gcttctccat ggccaccttt tcagcgatgt 900
atgcagctat ctggtcaacc tcctggacat tttttcagtc atataaaagc tatggtgaga 960
tgcagctgga aaagggtctt gggaaatatg aatgccccca gctggcccgt gacagactcc 1020
tgaggacagc tgtcctcttc tgcatcttgg ggacatctct ttgaattttc tgtgttttgc 1080
tgtaccagcc cagatgtttt acgtctggga gaaattgaca gatcaagctg tgagacagtg 1140
ggaaatattt agcaaataat ttcctggtgt gaaggtcctg ctattactaa ggagtaatct 1200
gtgtacaaag aaataacaag tcgatgaact attccccagc agggtctttt catctgggaa 1260
agacatccat aaagaagcaa taaagaagag tgccacattt atttttatat ctatatgtac 1320
ttgtcaaaga aggtttgtgt ttttctgctt ttgaaatctg tatctgtagt gagatagcat 1380
tgtgaactga caggcagcct ggacatagag agggagaaga agtcagagag ggtgacaaga 1440
tagagagcta tttaatggcc ggctggaaat gctgggctga cggtgcagtc tgggtgctcg 1500
cccacttgtc ccactatctg ggtgcatgat cttgagcaag ttccttctgg tgtctgcttt 1560
ctccattgta aaccacaagg ctgttgcatg ggctaatgaa gatcatatac gtgaaaatta 1620
tttgaaaaca tataaagcac tatacagatt cgaaactcca ttgagtcatt atccttgcta 1680
tgatgatggt gttttgggga tgagagggtg ctatccattt ctcatgtttt ccattgtttg 1740
aaacaaagaa ggttaccaag aagcctttcc tgtagccttc tgtaggaatt cttttgggga 1800
agtgaggaag ccaggtccac ggtctgttct tgaagcagta gcctaacaca ctccaagata 1860
tggacacacg ggagccgctg gcagaaggga cttcacgaag tgttgcatgg atgttttagc 1920
cattgttggc tttcccttat caaacttggg cccttccctt cttggtttcc aaaggcattt 1980
attgctgagt tatatgttca ctgtccccct aatattaggg agtaaaacgg ataccaagtt 2040

gatttagtgt ttttacctct gtcttggctt tcatgttatt aaacgtatgc atgtgaagaa 2100

gggtgttttt ctgttttata ttcaactcat aagactttgg gataggaaaa atgagtaatg 2160

gttactaggc ttaatacctg ggtgattaca taatctgtac aacgaacccc catgatgtaa 2220

gtttacctat gtaacaaacc tgcacttata cccatgaact taaaatgaaa gttaaaaata 2280

aaaaacatat acaaataaaa aaaa 2304

<210> 3
<211> 2574
<212> DNA
<213> homo sapiens

<220>
<221> mRNA
<222> (1)..(2574)
<223> CD83 mRNA, version NM_004233.2

<400> 3
cctggcgcag ccgcagcagc gacgcgagcg aactcggccg ggcccgggcg cgcgggggcg 60

ggacgcgcac gcggcgaggg cggcgggtga gccgggggcg gggacggggg cgggacgggg 120

gcgaaggggg cggggacggg ggcgcccgcc ggcctaacgg gattaggagg gcgcgccacc 180

cgcttccgct gcccgccggg gaatcccccg ggtggcgccc agggaagttc ccgaacgggc 240

gggcataaaa gggcagccgc gccggcgccc cacagctctg cagctcgtgg cagcggcgca 300

gcgctccagc catgtcgcgc ggcctccagc ttctgctcct gagctgcgcc tacagcctgg 360

ctcccgcgac gccggaggtg aaggtggctt gctccgaaga tgtggacttg ccctgcaccg 420

ccccctggga tccgcaggtt ccctacacgg tctcctgggt caagttattg gagggtggtg 480

aagagaggat ggagacaccc caggaagacc acctcagggg acagcactat catcagaagg 540

ggcaaaatgg ttctttcgac gcccccaatg aaaggcccta ttccctgaag atccgaaaca 600

ctaccagctg caactcgggg acatacaggt gcactctgca ggacccggat gggcagagaa 660

acctaagtgg caaggtgatc ttgagagtga caggatgccc tgcacagcgt aaagaagaga 720

cttttaagaa atacagagcg gagattgtcc tgctgctggc tctggttatt ttctacttaa 780

cactcatcat tttcacttgt aagtttgcac ggctacagag tatcttccca gatttttcta 840

aagctggcat ggaacgagct tttctcccag ttacctcccc aaataagcat ttagggctag 900

tgactcctca caagacagaa ctggtatgag caggatttct gcaggttctt cttcctgaag 960

ctgaggctca ggggtgtgcc tgtctgttac actggaggag agaagaatga gcctacgctg 1020

aagatggcat cctgtgaagt ccttcacctc actgaaaaca tctggaaggg gatcccaccc 1080

cattttctgt gggcaggcct cgaaaaccat cacatgacca catagcatga ggccactgct 1140

gcttctccat ggccaccttt tcagcgatgt atgcagctat ctggtcaacc tcctggacat 1200

tttttcagtc atataaaagc tatggtgaga tgcagctgga aaagggtctt gggaaatatg 1260

aatgccccca gctggcccgt gacagactcc tgaggacagc tgtcctcttc tgcatcttgg 1320

ggacatctct ttgaattttc tgtgttttgc tgtaccagcc cagatgtttt acgtctggga 1380

gaaattgaca gatcaagctg tgagacagtg ggaaatattt agcaaataat ttcctggtgt 1440

gaaggtcctg ctattactaa ggagtaatct gtgtacaaag aaataacaag tcgatgaact 1500

attccccagc agggtctttt catctgggaa agacatccat aaagaagcaa taaagaagag 1560

tgccacattt atttttatat ctatatgtac ttgtcaaaga aggtttgtgt ttttctgctt 1620

ttgaaatctg tatctgtagt gagatagcat tgtgaactga caggcagcct ggacatagag 1680

agggagaaga agtcagagag ggtgacaaga tagagagcta tttaatggcc ggctggaaat 1740

gctgggctga cggtgcagtc tgggtgctcg cccacttgtc ccactatctg ggtgcatgat 1800

cttgagcaag ttccttctgg tgtctgcttt ctccattgta aaccacaagg ctgttgcatg 1860

ggctaatgaa gatcatatac gtgaaaatta tttgaaaaca tataaagcac tatacagatt 1920

cgaaactcca ttgagtcatt atccttgcta tgatgatggt gttttgggga tgagagggtg 1980

ctatccattt ctcatgtttt ccattgtttg aaacaaagaa ggttaccaag aagcctttcc 2040

tgtagccttc tgtaggaatt cttttgggga agtgaggaag ccaggtccac ggtctgttct 2100

tgaagcagta gcctaacaca ctccaagata tggacacacg ggagccgctg gcagaaggga 2160

cttcacgaag tgttgcatgg atgttttagc cattgttggc tttcccttat caaacttggg 2220

cccttccctt cttggtttcc aaaggcattt attgctgagt tatatgttca ctgtccccct 2280

aatattaggg agtaaaacgg ataccaagtt gatttagtgt ttttacctct gtcttggctt 2340

tcatgttatt aaacgtatgc atgtgaagaa gggtgttttt ctgttttata ttcaactcat 2400

aagactttgg gataggaaaa atgagtaatg gttactaggc ttaatacctg ggtgattaca 2460

taatctgtac aacgaacccc catgatgtaa gtttacctat gtaacaaacc tgcacttata 2520

cccatgaact taaaatgaaa gttaaaaata aaaaacatat acaaataaaa aaaa 2574

<210> 4
<211> 21
<212> DNA
<213> artificial

<220>
<223> sense strand siRNA duplex #1

<400> 4
ugcuauuacu aaggaguaat t 21

<210> 5
<211> 21
<212> DNA

<213>  artificial

<220>
<223>  antisense strand siRNA duplex #1

<400>  5
uuacuccuua guaauagcag g                                                21


<210>  6
<211>  21
<212>  DNA
<213>  artificial

<220>
<223>  sense strand siRNA duplex #2

<400>  6
ggcauuuauu gcugaguuat t                                                21


<210>  7
<211>  21
<212>  DNA
<213>  artificial

<220>
<223>  antisense strand siRNA duplex #2

<400>  7
uaacucagca auaaaugcct t                                                21


<210>  8
<211>  21
<212>  DNA
<213>  artificial

<220>
<223>  sense strand siRNA duplex #3

<400>  8
ggaugggcag agaaaccuat t                                                21


<210>  9
<211>  21
<212>  DNA
<213>  artificial

<220>
<223>  antisense strand siRNA duplex #3

<400>  9
uagguuucuc ugcccauccg g                                                21


<210>  10
<211>  21
<212>  DNA
<213>  artificial

<220>

<223>  sense strand siRNA duplex #4

<400>  10
ggcuaaugaa gaucauauat t                                                21


<210>  11
<211>  21
<212>  DNA
<213>  artificial

<220>
<223>  antisense strand siRNA duplex #4

<400>  11
uauaugaucu ucauuagccc a                                                21


<210>  12
<211>  21
<212>  DNA
<213>  artificial

<220>
<223>  sense strand siRNA duplex #5

<400>  12
cggauaccaa guugauuuat t                                                21


<210>  13
<211>  21
<212>  DNA
<213>  artificial

<220>
<223>  antisense strand siRNA duplex #5

<400>  13
uaaaucaacu ugguauccgt t                                                21


<210>  14
<211>  20
<212>  DNA
<213>  artificial

<220>
<223>  sense (forward) primer CD80

<400>  14
ggacatgaat atatggcccg                                                  20


<210>  15
<211>  20
<212>  DNA
<213>  artificial

<220>
<223>  antisense (reverse) primer CD80

<400>  15

caacacactc gtatgtgccc                                                    20


<210>  16
<211>  20
<212>  DNA
<213>  artificial

<220>
<223>  sense (forward) primer CD83

<400>  16
cctccagctt ctgctcctga                                                    20


<210>  17
<211>  20
<212>  DNA
<213>  artificial

<220>
<223>  antisense (reverse) primer CD83

<400>  17
tcggagcaag ccaccttcac                                                    20


<210>  18
<211>  20
<212>  DNA
<213>  artificial

<220>
<223>  sense (forward) primer CD86

<400>  18
ttgcaaactc tcaaaaccaa                                                    20


<210>  19
<211>  20
<212>  DNA
<213>  artificial

<220>
<223>  antisense (reverse) primer CD86

<400>  19
ggaatgaaca ctgtcaaatt                                                    20


<210>  20
<211>  22
<212>  DNA
<213>  artificial

<220>
<223>  sense (forward) primer ß-glucuronidase

<400>  20
ctcatttgga attttgccga tt                                                 22

<210>  21
<211>  22
<212>  DNA
<213>  artificial

<220>
<223>  antisense (reverse) primer ß-glucuronidase

<400>  21
ccgagtgaag atcccctttt ta                                                22